# EUROPEAN PATENT SPECIFICATION

(11) **EP 4 313 396 B1**
(45) Date of publication and mention of the grant of the patent: **14.10.2026**
(21) Application number: 22718678.0
(22) Date of filing: 28.03.2022
(51) Int. Cl.: B01J 13/16, C11D 3/50, C11D 17/00

(54) **ACETOACETYL POLYMER-BASED MICROCAPSULES**
ACETOACETYLPOLYMERBASIERTE MIKROKAPSELN
MICROCAPSULES À BASE DE POLYMÈRE ACÉTOACÉTYLIQUE

(30) Priority: 30.03.2021 WO PCT/CN2021/083980; 07.05.2021 EP 21172800
(43) Date of publication of application: 07.02.2024
(73) Proprietor: Firmenich SA, 1242 Satigny (CH)
(72) Inventor: WU, Yongtao, Shanghai 201108 (CN); OUALI, Lahoussine, 1242 Satigny (CH); SUI, Xiaofeng, Shanghai 201620 (CN)
(74) Representative: dsm-firmenich IP
(86) International application number: PCT/EP2022/058079
(87) International publication number: WO 2022/207542

(56) References cited:
- WO-A1-2006/056093
- US-A1- 2016 158 121
- US-A1- 2019 021 962
- DATABASE WPI Week 199510, Derwent World Patents Index; AN 1995-069427, XP002804457

## Description

### Technical Field

The present invention relates to core-shell microcapsules, with a hydrophobic material-based core, preferably a perfume, and a polymeric shell comprising an acetoacetyl polymer. Process for preparing said microcapsules is also an object of the invention. Perfuming compositions and consumer products comprising said microcapsules, in particular perfumed consumer products in the form of home care or personal care products, are also part of the invention.

### Background of the Invention

One of the problems faced by the perfumery industry lies in the relatively rapid loss of olfactive benefit provided by odoriferous compounds due to their volatility, particularly that of "top-notes". In order to tailor the release rates of volatiles, delivery systems such as microcapsules containing a perfume, are needed to protect and later release the core payload when triggered. A key requirement from the industry regarding these systems is to survive suspension in challenging bases without physically dissociating or degrading. For instance, fragranced personal and household cleansers containing high levels of aggressive surfactant detergents are very challenging for the stability of microcapsules.

Aminoplast microcapsules formed of a melamine-formaldehyde resin have been largely used to encapsulate hydrophobic actives, thus protecting said actives and providing their controlled release. However, capsules such as aminoplast ones suffer from stability problems when used in consumer products comprising surfactants, such as perfumery consumer products, especially after prolonged storage at elevated temperatures. In such products, even though the capsule wall remains intact, the encapsulated active tends to leak out of the capsule by diffusion through the wall due to the presence of surfactants that are able to solubilise the encapsulated active in the product base. The leakage phenomenon reduces the efficiency of the capsules to protect the active and provide its controlled release.

A variety of strategies have been described to improve the stability of oil core-based microcapsules. Cross-linking of capsule walls, with chemical groups such as poly(amines) and poly(isocyanates), has been described as a way to improve stability of microcapsules. WO2011/154893 discloses for instance a process for the preparation of polyurea microcapsules using a combination of aromatic and aliphatic polyisocyanates in specific relative concentrations.

Stabilization of oil/water interfaces with inorganic particles has been described in so-called Pickering emulsions. In this context, functionalization of inorganic particles to allow their cross-linking is known. For instance, Pickering emulsions cross-linked from an outer water phase with polyelectrolytes providing electrostatic interactions have been the object of prior disclosures (Li Jian et al. in Langmuir (2010), 26(19), 15554-15560). However, such systems are very likely to dissociate in a surfactant base or in ethanol over time as electrostatic interactions are insufficient to promote stability. Covalent cross-linking has also been described in relation with Pickering emulsion in the preparation of colloidosomes. In particular, the use of diisocyanates as cross-linker has been disclosed in scientific publications. WO2009/063257 also describes the use of polyisocyanates as possible cross-linker for surface-modified inorganic particles in order to prepare microcapsules with increased level of protection from UV light for the contents. These products are typically intended for agrochemical applications. This type of system is not suitable for perfume encapsulation. In fact, in order to maintain a good morphology and permeability of the microcapsules, an excess of surface-modified inorganic particles is needed. Another problem is that these microcapsules show little margin for size adjustment. Furthermore, the amount of adsorbed particles at the oil-water interface is limited which affects the properties of the capsule membranes.

Moreover, in addition to the performance in terms of stability and olfactive performance, the consumer demand for eco-friendly delivery systems is more and more important and is driving the development of new delivery systems.

JP H06 343852 A discloses a polyurea microcapsule comprising an oil-based core. Since an acetoacetyl group-modified polyvinyl alcohol was present as an emulsifier in the aqueous phase during the preparation of the microcapsule, it is unavoidable that the shell of the microcapsule must comprise said acetoacetyl group-modified polyvinyl alcohol. The microcapsule may comprise a perfume.

There is therefore still a need to provide new microcapsules using more eco-friendly materials, while not compromising on the performance of the microcapsules, in particular in terms of stability in a challenging medium such as a consumer product base, as well as in delivering a good performance in terms of active ingredient delivery, e.g. olfactive performance in the case of perfuming ingredients.

### Summary of the Invention

A first aspect of the invention is therefore a core-shell microcapsule comprising:
a) an oil-based core comprising a hydrophobic material, preferably a perfume oil; and
b) a polymeric shell comprising an acetoacetyl polymer, wherein the acetoacetyl polymer is not an acetoacetyl polyvinyl alcohol (PVOH) or an acetoacetyl-modified polyvinyl alcohol (PVOH).

Also disclosed is a core-shell microcapsule slurry comprising at least a microcapsule made of:
a) an oil-based core comprising a hydrophobic material, preferably a perfume oil; and
b) a polymeric shell comprising an acetoacetyl polymer.

Another aspect of the invention is a process for preparing core-shell microcapsules as defined above, wherein the process comprises the steps of:
1) preparing a water phase;
2) preparing an oil phase comprising a hydrophobic material, preferably a perfume oil;
3) adding the oil phase to the water phase and mixing them to form an oil-in-water emulsion, under conditions allowing the formation of core-shell microcapsule by interfacial polymerization and/or interfacial reaction,

wherein a polyfunctional monomer is added in step 1) in the water phase and/or in step 2) in the oil phase and/or in step 3) in the emulsion, and
wherein an acetoacetyl polymer is added in the water phase and/or in the oil phase and/or in the emulsion.

Also disclosed is a microcapsule obtainable by such a process as well as perfuming compositions and consumer products containing them.

Also disclosed is the use of an acetoacetyl polymer, for the stabilization of an emulsion and/or further subjected to an interfacial polymerisation and/or interfacial reaction.

The invention is as defined in the appended claims.

### Detailed Description of the Invention.

Unless stated otherwise, percentages (%) are meant to designate percent by weight of a composition.

By "**hydrophobic material**", it is meant a material which forms a two-phase dispersion when mixed with water. According to the invention, the hydrophobic material can be "inert" material like solvents or active ingredients. According to an embodiment, the hydrophobic material is a hydrophobic active ingredient.

By "**active ingredient**", it is meant a single compound or a combination of ingredients.

By "**perfume oil**", it is meant a single perfuming or a mixture of several perfuming compounds.

By "**consumer product**" or "**end-product**" it is meant a manufactured product ready to be distributed, sold and used by a consumer.

A "**microcapsule**", or the similar, in the present invention has a morphology that can vary from a core-shell to a matrix type. According to one embodiment, it is of the core-shell type. In this case, the microcapsules comprise a core based on a hydrophobic material, typically a perfume, and a polymeric shell comprising an acetoacetyl polymer.

Typically, microcapsules have a microcapsule size distribution in the micron range (e.g. a mean diameter) comprised between about 1 and 3000 microns, preferably comprised between 1 and 1000 microns, more preferably between 1 and 500 microns, and even more preferably between 5 and 50 microns. The polymeric shell of the microcapsule according to the present invention is formed by interfacial polymerization and/or interfacial reaction in the presence of an acetoacetyl polymer. More particularly, the polymeric shell is formed by a reaction between a polyfunctional monomer, and optionally a reactant, in the presence of the acetoacetyl polymer. The acetoacetyl polymer can participate to the polymeric shell formation and/or interact with the polymeric shell.

By "**microcapsule slurry**", it is meant microcapsule(s) that is (are) dispersed in a liquid. According to an embodiment, the microcapsule(s) is (are) dispersed in water.

By "**particle size**" it is meant an average diameter of particles based on size distribution measured by dynamic light scattering (DLS) using Zetasizer Nano ZS equipment from Malvern Instruments Ltd., UK when particles are dispersed into a water phase.

By "**microcapsules size**" it is meant the volume mean diameter (D[4,3]) of the relevant microcapsules, microcapsules suspension as obtained by laser light scattering of a diluted sample in a Malvern Mastersizer 3000.

By "**an acetoacetyl polymer**", it is meant a polymer having at least two acetoacetate functional groups. The acetoacetyl polymer can be used as a single acetoacetyl polymer or as a mixture of acetoacetyl polymer.

Acetoacetyl polymer used in the present invention can be obtained by an acetoacetylation of a polymer substrate (or polymer) containing hydroxyl groups. In the present invention, "a polymer substrate containing hydroxyl groups" or "a polymer containing hydroxyl groups" are used indifferently.

As non-limiting example of polymer substrate, we can cite polysaccharides (such as cellulose, starch, Gum Arabic, chitosan), synthetic polymer (such as PEG (polyethylene glycol), or polyols. Then, the acetoacetylation can be obtained by
(1) a reaction of corresponding hydroxy compounds with diketene or diketene derivatives; or
(2) a transesterification of corresponding hydroxy compounds with acetoacetate.

According to the invention, by "the polymeric shell comprising an acetoacetyl polymer", it should be understood that the polymeric shell comprises an acetoacetyl polymer and/or a material derived from an acetoacetyl polymer.

By "**polyfunctional monomer**", it is meant a molecule that, as unit, reacts or binds chemically to form a polymer or a supramolecular polymer. The polyfunctional monomer is oil soluble or water soluble. The polyfunctional monomer of the invention has at least two functional groups that are capable to react with or bind to functional groups of another component (for example acetoacetyl polymer) and/or are capable to polymerize to form a polymeric shell. The wording "shell" and "wall" are used indifferently in the present invention.

By "**polyurea**" wall or shell, it is meant that the polymeric shell comprises urea linkages produced by either an amino-functional crosslinker or hydrolysis of isocyanate groups to produce amino groups capable of further reacting with isocyanate groups during interfacial polymerization. According to a particular embodiment, polyurea-based capsules are formed in the absence of added amine reactant.

It has now surprisingly been found that performing core-shell microcapsules encapsulating a hydrophobic material, for example a perfume oil, could be obtained when an acetoacetyl polymer is comprised within the shell. The microcapsules of the invention therefore provide a solution to the above-mentioned problems as it improves the storage stability in challenging bases even with a low concentration of polymeric material in the shell.

### CORE-SHELL MICROCAPSULE

A first object of the invention is a core-shell microcapsule comprising:
a) an oil-based core comprising a hydrophobic material, preferably a perfume oil; and
b) a polymeric shell comprising an acetoacetyl polymer, wherein the acetoacetyl polymer is not an acetoacetyl polyvinyl alcohol (PVOH) or an acetoacetyl-modified polyvinyl alcohol (PVOH).

According to an embodiment, the polymeric shell is formed by interfacial reaction/polymerisation in the presence of an acetoacetyl polymer.

### Hydrophobic material

The hydrophobic material according to the invention can be "inert" material like solvents or active ingredients.

When hydrophobic materials are active ingredients, they are preferably chosen from the group consisting of flavors, flavor ingredients, perfumes, perfume ingredients, nutraceuticals, cosmetics, pest control agents, biocide actives, malodor counteracting ingredient and mixtures thereof.

According to a particular embodiment, the hydrophobic material is not a phase change material (PCM).

According to a particular embodiment, the hydrophobic material comprises a mixture of a perfume with another ingredient selected from the group consisting of nutraceuticals, cosmetics, pest control agents and biocide actives.

According to a particular embodiment, the hydrophobic material comprises a mixture of biocide actives with another ingredient selected from the group consisting of perfumes, nutraceuticals, cosmetics, pest control agents.

According to a particular embodiment, the hydrophobic material comprises a mixture of pest control agents with another ingredient selected from the group consisting of perfumes, nutraceuticals, cosmetics, biocide actives.

According to a particular embodiment, the hydrophobic material comprises a perfume.

According to a particular embodiment, the hydrophobic material consists of a perfume.

According to a particular embodiment, the hydrophobic material consists of biocide actives.

According to a particular embodiment, the hydrophobic material consists of pest control agents.

By "perfume" (or also "perfume oil") what is meant here is an ingredient or a composition that is a liquid at about 20°C. According to any one of the above embodiments said perfume oil can be a perfuming ingredient alone or a mixture of ingredients in the form of a perfuming composition. As a "perfuming ingredient" it is meant here a compound, which is used for the primary purpose of conferring or modulating an odor. In other words such an ingredient, to be considered as being a perfuming one, must be recognized by a person skilled in the art as being able to at least impart or modify in a positive or pleasant way the odor of a composition, and not just as having an odor. For the purpose of the present invention, perfume oil also includes a combination of perfuming ingredients with substances which together improve, enhance or modify the delivery of the perfuming ingredients, such as perfume precursors, modulators, emulsions or dispersions, as well as combinations which impart an additional benefit beyond that of modifying or imparting an odor, such as long-lastingness, blooming, malodor counteraction, antimicrobial effect, microbial stability, pest control.

The nature and type of the perfuming ingredients present in the oil phase do not warrant a more detailed description here, which in any case would not be exhaustive, the skilled person being able to select them on the basis of its general knowledge and according to intended use or application and the desired organoleptic effect. In general terms, these perfuming ingredients belong to chemical classes as varied as alcohols, aldehydes, ketones, esters, ethers, acetates, nitriles, terpenoids, nitrogenous or sulfurous heterocyclic compounds and essential oils (for example Thyme oil), and said perfuming co-ingredients can be of natural or synthetic origin. Many of these co-ingredients are in any case listed in reference texts such as the book by S. Arctander, Perfume and Flavor Chemicals, 1969, Montclair, New Jersey, USA, or its more recent versions, or in other works of a similar nature, as well as in the abundant patent literature in the field of perfumery.

In particular one may cite perfuming ingredients which are commonly used in perfume formulations, such as:
- Aldehydic ingredients: decanal, dodecanal, 2-methyl-undecanal, 10-undecenal, octanal, nonanal and/or nonenal;
- Aromatic-herbal ingredients: eucalyptus oil, camphor, eucalyptol, 5-methyltricyclo[6.2.1.0^{2,7}]undecan-4-one, 1-methoxy-3-hexanethiol, 2-ethyl-4,4-dimethyl-1,3-oxathiane, 2,2,7/8,9/10-tetramethylspiro[5.5]undec-8-en-1-one, menthol and/or alpha-pinene;
- Balsamic ingredients: coumarin, ethylvanillin and/or vanillin;
- Citrus ingredients: dihydromyrcenol, citral, orange oil, linalyl acetate, citronellyl nitrile, orange terpenes, limonene, 1-p-menthen-8-yl acetate and/or 1,4(8)-p-menthadiene;
- Floral ingredients: methyl dihydrojasmonate, linalool, citronellol, phenylethanol, 3-(4-tert-butylphenyl)-2-methylpropanal, hexylcinnamic aldehyde, benzyl acetate, benzyl salicylate, tetrahydro-2-isobutyl-4-methyl-4(2H)-pyranol, beta ionone, methyl 2-(methylamino)benzoate, (E)-3-methyl-4-(2,6,6-trimethyl-2-cyclohexen-1-yl)-3-buten-2-one, (1E)-1-(2,6,6-trimethyl-2-cyclohexen-1-yl)-1-penten-3-one, 1-(2,6,6-trimethyl-1,3-cyclohexadien-1-yl)-2-buten-1-one, (2E)-1-(2,6,6-trimethyl-2-cyclohexen-1-yl)-2-buten-1-one, (2E)-1-[2,6,6-trimethyl-3-cyclohexen-1-yl]-2-buten-1-one, (2E)-1-(2,6,6-trimethyl-1-cyclohexen-1-yl)-2-buten-1-one, 3-(3,3/1,1-dimethyl-5-indanyl)propanal, 2,5-dimethyl-2-indanmethanol, 2,6,6-trimethyl-3-cyclohexene-1-carboxylate, 3-(4,4-dimethyl-1-cyclohexen-1-yl)propanal, hexyl salicylate, 3,7-dimethyl-1,6-nonadien-3-ol, 3-(4-isopropylphenyl)-2-methylpropanal, verdyl acetate, geraniol, p-menth-1-en-8-ol, 4-(1,1-dimethylethyl)-1-cyclohexyle acetate, 1,1-dimethyl-2-phenylethyl acetate, 4-cyclohexyl-2-methyl-2-butanol, amyl salicylate , high cis methyl dihydrojasmonate, 3-methyl-5-phenyl-1-pentanol, verdyl proprionate, geranyl acetate, tetrahydro linalool, cis-7-p-menthanol, propyl (S)-2-(1,1-dimethylpropoxy)propanoate, 2-methoxynaphthalene, 2,2,2-trichloro-1-phenylethyl acetate, 4/3-(4-hydroxy-4-methylpentyl)-3-cyclohexene-1-carbaldehyde, amylcinnamic aldehyde, 8-decen-5-olide, 4-phenyl-2-butanone, isononyle acetate, 4-(1,1-dimethylethyl)-1-cyclohexyl acetate, verdyl isobutyrate and/or mixture of methylionones isomers;
- Fruity ingredients: gamma-undecalactone, 2,2,5-trimethyl-5-pentylcyclopentanone, 2-methyl-4-propyl-1,3-oxathiane, 4-decanolide, ethyl 2-methyl-pentanoate, hexyl acetate, ethyl 2-methylbutanoate, gamma-nonalactone, allyl heptanoate, 2-phenoxyethyl isobutyrate, ethyl 2-methyl-1,3-dioxolane-2-acetate, diethyl 1,4-cyclohexanedicarboxylate, 3-methyl-2-hexen-1-yl acetate, 1-[3,3-dimethylcyclohexyl]ethyl [3-ethyl-2-oxiranyl]acetate and/or diethyl 1,4-cyclohexane dicarboxylate;
- Green ingredients: 2-methyl-3-hexanone (E)-oxime, 2,4-dimethyl-3-cyclohexene-1-carbaldehyde, 2-tert-butyl-1-cyclohexyl acetate, styrallyl acetate, allyl (2-methylbutoxy)acetate, 4-methyl-3-decen-5-ol, diphenyl ether, (Z)-3-hexen-1-ol and/or 1-(5,5-dimethyl-1-cyclohexen-1-yl)-4-penten-1-one;
- Musk ingredients: 1,4-dioxa-5,17-cycloheptadecanedione, (Z)-4-cyclopentadecen-1-one, 3-methylcyclopentadecanone, 1-oxa-12-cyclohexadecen-2-one, 1-oxa-13-cyclohexadecen-2-one, (9Z)-9-cycloheptadecen-1-one, 2-{(1S)-1-[(1R)-3,3-dimethylcyclohexyl]ethoxy}-2-oxoethyl propionate, 3-methyl-5-cyclopentadecen-1-one, 4,6,6,7,8,8-hexamethyl-1,3,4,6,7,8-hexahydrocyclopenta[g]isochromene, (1S,1'R)-2-[1-(3',3'-dimethyl-1'-cyclohexyl)ethoxy]-2-methylpropyl propanoate, oxacyclohexadecan-2-one and/or (1S,1'R)-[1-(3',3'-dimethyl-1'-cyclohexyl)ethoxycarbonyl]methyl propanoate;
- Woody ingredients: 1-[(1RS,6SR)-2,2,6-trimethylcyclohexyl]-3-hexanol, 3,3-dimethyl-5-[(1R)-2,2,3-trimethyl-3-cyclopenten-1-yl]-4-penten-2-ol, 3,4'-dimethylspiro[oxirane-2,9'-tricyclo[6.2.1.0^{2,7}]undec[4]ene, (1-ethoxyethoxy)cyclododecane, 2,2,9,11-tetramethylspiro[5.5]undec-8-en-1-yl acetate, 1-(octahydro-2,3,8,8-tetramethyl-2-naphtalenyl)-1-ethanone, patchouli oil, terpenes fractions of patchouli oil, Clearwood^{®}, (1'R,E)-2-ethyl-4-(2',2',3'-trimethyl-3'-cyclopenten-1'-yl)-2-buten-1-ol, 2-ethyl-4-(2,2,3-trimethyl-3-cyclopenten-1-yl)-2-buten-1-ol, methyl cedryl ketone, 5-(2,2,3-trimethyl-3-cyclopentenyl)-3 -methylpentan-2-ol, 1-(2,3,8,8-tetramethyl-1,2,3,4,6,7,8,8a-octahydronaphthalen-2-yl)ethan-1-one and/or isobornyl acetate;
- Other ingredients (e.g. amber, powdery spicy or watery): dodecahydro-3a,6,6,9a-tetramethyl-naphtho[2,1-b]furan and any of its stereoisomers, heliotropin, anisic aldehyde, eugenol, cinnamic aldehyde, clove oil, 3-(1,3-benzodioxol-5-yl)-2-methylpropanal, 7-methyl-2H-1,5-benzodioxepin-3(4H)-one, 2,5,5-trimethyl-1,2,3,4,4a,5,6,7-octahydro-2-naphthalenol, 1-phenylvinyl acetate, 6-methyl-7-oxa-1-thia-4-azaspiro[4.4]nonane and/or 3-(3-isopropyl-1-phenyl)butanal.

It is also understood that said ingredients may also be compounds known to release in a controlled manner various types of perfuming compounds also known as properfume or profragrance. Non-limiting examples of suitable properfumes may include 4-(dodecylthio)-4-(2,6,6-trimethyl-2-cyclohexen-1-yl)-2-butanone, 4-(dodecylthio)-4-(2,6,6-trimethyl-1-cyclohexen-1-yl)-2-butanone, 3-(dodecylthio)-1-(2,6,6-trimethyl-3-cyclohexen-1-yl)-1-butanone, 2-(dodecylthio)octan-4-one, 2-phenylethyl oxo(phenyl)acetate, 3,7-dimethylocta-2,6-dien-1-yl oxo(phenyl)acetate, (Z)-hex-3-en-1-yl oxo(phenyl)acetate, 3,7-dimethyl-2,6-octadien-1-yl hexadecanoate, bis(3,7-dimethylocta-2,6-dien-1-yl) succinate, (2-((2-methylundec-1-en-1-yl)oxy)ethyl)benzene, 1-methoxy-4-(3-methyl-4-phenethoxybut-3-en-1-yl)benzene, (3-methyl-4-phenethoxybut-3-en-1-yl)benzene, 1-(((Z)-hex-3-en-1-yl)oxy)-2-methylundec-1-ene, (2-((2-methylundec-1-en-1-yl)oxy)ethoxy)benzene, 2-methyl-1-(octan-3-yloxy)undec-1-ene, 1-methoxy-4-(1-phenethoxyprop-1-en-2-yl)benzene, 1-methyl-4-(1-phenethoxyprop-1-en-2-yl)benzene, 2-(1-phenethoxyprop-1-en-2-yl)naphthalene, (2-phenethoxyvinyl)benzene, 2-(1-((3,7-dimethyloct-6-en-1-yl)oxy)prop-1-en-2-yl)naphthalene, (2-((2-pentylcyclopentylidene)methoxy)ethyl)benzene, 4-allyl-2-methoxy-1-((2-methoxy-2-phenylvinyl)oxy)benzene, (2-((2-heptylcyclopentylidene)methoxy)ethyl)benzene, 1-isopropyl-4-methyl-2-((2-pentylcyclopentylidene)methoxy)benzene, 2-methoxy-1-((2-pentylcyclopentylidene)methoxy)-4-propylbenzene, 3-methoxy-4-((2-methoxy-2-phenylvinyl)oxy)benzaldehyde, 4-((2-(hexyloxy)-2-phenylvinyl)oxy)-3-methoxybenzaldehyde or a mixture thereof.

The perfuming ingredients may be dissolved in a solvent of current use in the perfume industry. The solvent is preferably not an alcohol. Examples of such solvents are diethyl phthalate, isopropyl myristate, Abalyn^{®} (rosin resins, available from Eastman), benzyl benzoate, ethyl citrate, triethyl citrate, limonene or other terpenes, or isoparaffins. Preferably, the solvent is very hydrophobic and highly sterically hindered, like for example Abalyn^{®} or benzyl benzoate. Preferably the perfume comprises less than 30% of solvent. More preferably the perfume comprises less than 20% and even more preferably less than 10% of solvent, all these percentages being defined by weight relative to the total weight of the perfume. Most preferably, the perfume is essentially free of solvent.

Preferred perfuming ingredients are those having a high steric hindrance (i.e bulky materials) and in particular those from one of the following groups:
- Group 1: perfuming ingredients comprising a cyclohexane, cyclohexene, cyclohexanone or cyclohexenone ring substituted with at least one linear or branched C₁ to C₄ alkyl or alkenyl substituent;
- Group 2: perfuming ingredients comprising a cyclopentane, cyclopentene, cyclopentanone or cyclopentenone ring substituted with at least one linear or branched C₄ to C₈ alkyl or alkenyl substituent;
- Group 3: perfuming ingredients comprising a phenyl ring or perfuming ingredients comprising a cyclohexane, cyclohexene, cyclohexanone or cyclohexenone ring substituted with at least one linear or branched C₅ to C₈ alkyl or alkenyl substituent or with at least one phenyl substituent and optionally one or more linear or branched C₁ to C₃ alkyl or alkenyl substituents;
- Group 4: perfuming ingredients comprising at least two fused or linked C₅ and/or C₆ rings;
- Group 5: perfuming ingredients comprising a camphor-like ring structure;
- Group 6: perfuming ingredients comprising at least one C₇ to C₂₀ ring structure;
- Group 7: perfuming ingredients having a logP value above 3.5 and comprising at least one tert-butyl or at least one trichloromethyl substitutent;

Examples of ingredients from each of these groups are:
- Group 1: 2,4-dimethyl-3-cyclohexene-1-carbaldehyde (origin: Firmenich SA, Geneva, Switzerland), isocyclocitral, menthone, isomenthone, methyl 2,2-dimethyl-6-methylene-1-cyclohexanecarboxylate (origin: Firmenich SA, Geneva, Switzerland), nerone, terpineol, dihydroterpineol, terpenyl acetate, dihydroterpenyl acetate, dipentene, eucalyptol, hexylate, rose oxide, (S)-1,8-p-menthadiene-7-ol (origin: Firmenich SA, Geneva, Switzerland), 1-p-menthene-4-ol, (1RS,3RS,4SR)-3-p-mentanyl acetate, (1R,2S,4R)-4,6,6-trimethyl-bicyclo[3,1,1]heptan-2-ol, tetrahydro-4-methyl-2-phenyl-2H-pyran (origin: Firmenich SA, Geneva, Switzerland), cyclohexyl acetate, cyclanol acetate, 1,4-cyclohexane diethyldicarboxylate (origin: Firmenich SA, Geneva, Switzerland), (3RS,3aRS,6SR,7ASR)-perhydro-3,6-dimethyl-benzo[B]furan-2-one (origin: Firmenich SA, Geneva, Switzerland), ((6R)-perhydro-3,6-dimethyl-benzo[B]furan-2-one (origin: Firmenich SA, Geneva, Switzerland), 2,4,6-trimethyl-4-phenyl-1,3-dioxane, 2,4,6-trimethyl-3-cyclohexene-1-carbaldehyde;
- Group 2: (E)-3-methyl-5-(2,2,3-trimethyl-3-cyclopenten-1-yl)-4-penten-2-ol (origin: Givaudan SA, Vernier, Switzerland), (1'R,E)-2-ethyl-4-(2',2',3'-trimethyl-3'-cyclopenten-1'-yl)-2-buten-1-ol (origin: Firmenich SA, Geneva, Switzerland), (1'R,E)-3,3-dimethyl-5-(2',2',3'-trimethyl-3'-cyclopenten-1'-yl)-4-penten-2-ol (origin: Firmenich SA, Geneva, Switzerland), 2-heptylcyclopentanone, methyl-cis-3-oxo-2-pentyl-1-cyclopentane acetate (origin: Firmenich SA, Geneva, Switzerland), 2,2,5-trimethyl-5-pentyl-1-cyclopentanone (origin: Firmenich SA, Geneva, Switzerland), 3,3-dimethyl-5-(2,2,3-trimethyl-3-cyclopenten-1-yl)-4-penten-2-ol (origin: Firmenich SA, Geneva, Switzerland), 3-methyl-5-(2,2,3-trimethyl-3-cyclopenten-1-yl)-2-pentanol (origin, Givaudan SA, Vernier, Switzerland);
- Group 3: damascones, 1-(5,5-dimethyl-1-cyclohexen-1-yl)-4-penten-1-one (origin: Firmenich SA, Geneva, Switzerland), (1'R)-2-[2-(4'-methyl-3'-cyclohexen-1'-yl)propyl]cyclopentanone, alpha-ionone, beta-ionone, damascenone, mixture of 1-(5,5-dimethyl-1-cyclohexen-1-yl)-4-penten-1-one and 1-(3,3-dimethyl-1-cyclohexen-1-yl)-4-penten-1-one (origin: Firmenich SA, Geneva, Switzerland), 1-(2,6,6-trimethyl-1-cyclohexen-1-yl)-2-buten-1-one (origin: Firmenich SA, Geneva, Switzerland), (1S,1'R)-[1-(3',3'-Dimethyl-1'-cyclohexyl)ethoxycarbonyl]methyl propanoate (origin: Firmenich SA, Geneva, Switzerland), 2-tert-butyl-1-cyclohexyl acetate (origin: International Flavors and Fragrances, USA), 1-(2,2,3,6-tetramethyl-cyclohexyl)-3-hexanol (origin: Firmenich SA, Geneva, Switzerland), trans-1-(2,2,6-trimethyl-1-cyclohexyl)-3-hexanol (origin: Firmenich SA, Geneva, Switzerland), (E)-3-methyl-4-(2,6,6-trimethyl-2-cyclohexen-1-yl)-3-buten-2-one, terpenyl isobutyrate, 4-(1,1-dimethylethyl)-1-cyclohexyl acetate (origin: Firmenich SA, Geneva, Switzerland), 8-methoxy-1-p-menthene, (1S,1R)-2-[1-(3',3'-dimethyl-1'-cyclohexyl) ethoxy]-2-methylpropyl propanoate (origin: Firmenich SA, Geneva, Switzerland), para tert-butylcyclohexanone, menthenethiol, 1-methyl-4-(4-methyl-3-pentenyl)-3-cyclohexene-1-carbaldehyde, allyl cyclohexylpropionate, cyclohexyl salicylate, 2-methoxy-4-methylphenyl methyl carbonate, ethyl 2-methoxy-4-methylphenyl carbonate, 4-ethyl-2-methoxyphenyl methyl carbonate;
- Group 4: Methyl cedryl ketone (origin: International Flavors and Fragrances, USA), a mixture of (1RS,2SR,6RS,7RS,8SR)-tricyclo[5.2.1.0^{2,6}]dec-3-en-8-yl 2-methylpropanoate and (1RS,2SR,6RS, 7RS,8SR)-tricyclo[5.2.1.0^{2,6}]dec-4-en-8-yl 2-methylpropanoate, vetyverol, vetyverone, 1-(octahydro-2,3,8,8-tetramethyl-2-naphtalenyl)-1-ethanone (origin: International Flavors and Fragrances, USA), (5RS,9RS,10SR)-2,6,9,10-tetramethyl-1-oxaspiro[4.5]deca-3,6-diene and the (5RS,9SR,10RS) isomer, 6-ethyl-2,10,10-trimethyl-1-oxaspiro[4.5]deca-3,6-diene, 1,2,3,5,6,7-hexahydro-1,1,2,3,3-pentamethyl-4-indenone (origin: International Flavors and Fragrances, USA), a mixture of 3-(3,3-dimethyl-5-indanyl)propanal and 3-(1,1-dimethyl-5-indanyl)propanal (origin: Firmenich SA, Geneva, Switzerland), 3',4-dimethyl-tricyclo[6.2.1.0(2,7)]undec-4-ene-9-spiro-2'-oxirane (origin: Firmenich SA, Geneva, Switzerland), 9/10-ethyldiene-3-oxatricyclo[6.2.1.0(2,7)]undecane, (perhydro-5,5,8A-trimethyl-2-naphthalenyl acetate (origin: Firmenich SA, Geneva, Switzerland), octalynol, (dodecahydro-3a,6,6,9a-tetramethyl-naphtho[2,1-b]furan, origin: Firmenich SA, Geneva, Switzerland), tricyclo[5.2.1.0(2,6)]dec-3-en-8-yl acetate and tricyclo[5.2.1.0(2,6)]dec-4-en-8-yl acetate as well as tricyclo[5.2.1.0(2,6)]dec-3-en-8-yl propanoate and tricyclo[5.2.1.0(2,6)]dec-4-en-8-yl propanoate, (+)-(1S,2S,3S)-2,6,6-trimethyl-bicyclo[3.1.1]heptane-3-spiro-2'-cyclohexen-4'-one;
- Group 5: camphor, borneol, isobornyl acetate, 8-isopropyl-6-methyl-bicyclo[2.2.2]oct-5-ene-2-carbaldehyde, pinene, camphene, 8-methoxycedrane, (8-methoxy-2,6,6,8-tetramethyl-tricyclo[5.3.1.0(1,5)]undecane (origin: Firmenich SA, Geneva, Switzerland), cedrene, cedrenol, cedrol, mixture of 9-ethylidene-3-oxatricyclo[6.2.1.0(2,7)]undecan-4-one and 10-ethylidene-3-oxatricyclo[6.2.1.0^{2,7}]undecan-4-one (origin: Firmenich SA, Geneva, Switzerland), 3-methoxy-7,7-dimethyl-10-methylene-bicyclo[4.3.1]decane (origin: Firmenich SA, Geneva, Switzerland);
- Group 6: (trimethyl-13-oxabicyclo-[10.1.0]-trideca-4,8-diene (origin: Firmenich SA, Geneva, Switzerland), 9-hexadecen-16-olide (origin: Firmenich SA, Geneva, Switzerland), pentadecenolide (origin: Firmenich SA, Geneva, Switzerland), 3-methyl-(4/5)-cyclopentadecenone ,(origin: Firmenich SA, Geneva, Switzerland), 3-methylcyclopentadecanone (origin: Firmenich SA, Geneva, Switzerland), pentadecanolide (origin: Firmenich SA, Geneva, Switzerland), cyclopentadecanone (origin: Firmenich SA, Geneva, Switzerland), 1-ethoxyethoxy)cyclododecane (origin: Firmenich SA, Geneva, Switzerland), 1,4-dioxacycloheptadecane-5,17-dione, 4,8-cyclododecadien-1-one;
- Group 7: (+-)-2-methyl-3-[4-(2-methyl-2-propanyl)phenyl]propanal (origin: Givaudan SA, Vernier, Switzerland), 2,2,2-trichloro-1-phenylethyl acetate.

Preferably, the perfume comprises at least 30%, preferably at least 50%, more preferably at least 60% of ingredients selected from Groups 1 to 7, as defined above. More preferably said perfume comprises at least 30%, preferably at least 50% of ingredients from Groups 3 to 7, as defined above. Most preferably said perfume comprises at least 30%, preferably at least 50% of ingredients from Groups 3, 4, 6 or 7, as defined above.

According to another preferred embodiment, the perfume comprises at least 30%, preferably at least 50%, more preferably at least 60% of ingredients having a logP above 3, preferably above 3.5 and even more preferably above 3.75.

According to a particular embodiment, the perfume used in the invention contains less than 10% of its own weight of primary alcohols, less than 15% of its own weight of secondary alcohols and less than 20% of its own weight of tertiary alcohols. Advantageously, the perfume used in the invention does not contain any primary alcohols and contains less than 15% of secondary and tertiary alcohols.

According to an embodiment, the oil phase (or the oil-based core) comprises:
- 25-100wt% of a perfume oil comprising at least 15wt% of high impact perfume raw materials having a Log T<-4, and
- 0-75wt% of a density balancing material having a density greater than 1.07 g/cm³. "*High impact perfume raw materials*" should be understood as perfume raw materials having a LogT<-4. The odor threshold concentration of a chemical compound is determined in part by its shape, polarity, partial charges and molecular mass. For convenience, the odor threshold concentration is presented as the common logarithm of the threshold concentration, i.e., Log [Threshold] ("LogT").

A "*density balancing material*" should be understood as a material having a density greater than 1.07 g/cm³ and having preferably low or no odor.

The odor threshold concentration of a perfuming compound is determined by using a gas chromatograph ("GC"). Specifically, the gas chromatograph is calibrated to determine the exact volume of the perfume oil ingredient injected by the syringe, the precise split ratio, and the hydrocarbon response using a hydrocarbon standard of known concentration and chain-length distribution. The air flow rate is accurately measured and, assuming the duration of a human inhalation to last 12 seconds, the sampled volume is calculated. Since the precise concentration at the detector at any point in time is known, the mass per volume inhaled is known and hence the concentration of the perfuming compound. To determine the threshold concentration, solutions are delivered to the sniff port at the back-calculated concentration. A panelist sniffs the GC effluent and identifies the retention time when odor is noticed. The average across all panelists determines the odor threshold concentration of the perfuming compound. The determination of odor threshold is described in more detail in C. Vuilleumier et al., Multidimensional Visualization of Physical and Perceptual Data Leading to a Creative Approach in Fragrance Development, Perfume & Flavorist, Vol. 33, September,, 2008, pages 54-61.

The nature of high impact perfume raw materials having a Log T<-4 and density balancing material having a density greater than 1.07 g/cm³ are described in WO2018115250.

According to an embodiment, the high impact perfume raw materials having a Log T<-4 are selected from the group consisting of (+-)-1-methoxy-3-hexanethiol, 4-(4-hydroxy-1-phenyl)-2-butanone, 2-methoxy-4-(1-propenyl)-1-phenyl acetate, pyrazobutyle, 3-propylphenol, 1-(3-methyl-1-benzofuran-2-yl)ethanone, 2-(3-phenylpropyl)pyridine, 1-(3,3/5,5-dimethyl-1-cyclohexen-1-yl)-4-penten-1-one , 1-(5,5-dimethyl-1-cyclohexen-1-yl)-4-penten-1-one, a mixture comprising (3RS,3aRS,6SR,7ASR)-perhydro-3,6-dimethyl-benzo[b]furan-2-one and (3SR,3aRS,6SR,7ASR)-perhydro-3,6-dimethyl-benzo[b]furan-2-one, (+-)-1-(5-ethyl-5-methyl-1-cyclohexen-1-yl)-4-penten-1-one, (1'S,3'R)-1-methyl-2-[(1',2',2'-trimethylbicyclo[3.1.0]hex-3'-yl)methyl]cyclopropyl}methanol, (+-)-3-mercaptohexyl acetate, (2E)-1-(2,6,6-trimethyl-1,3-cyclohexadien-1-yl)-2-buten-1-one, H-methyl-2h-1,5-benzodioxepin-3(4H)-one, (2E,6Z)-2,6-nonadien-1-ol, (4Z)-4-dodecenal, (+-)-4-hydroxy-2,5-dimethyl-3(2H)-furanone, methyl 2,4-dihydroxy-3,6-dimethylbenzoate, 3-methylindole, (+-)-perhydro-4alpha,8abeta-dimethyl-4a-naphthalenol, patchoulol, 2-methoxy-4-(1-propenyl)phenol, mixture comprising (+-)-5,6-dihydro-4-methyl-2-phenyl-2H-pyran and tetrahydro-4-methylene-2-phenyl-2H-pyran, mixture comprising 4-methylene-2-phenyltetrahydro-2H-pyran and (+-)-4-methyl-2-phenyl-3,6-dihydro-2H-pyran, 4-hydroxy-3-methoxybenzaldehyde, nonylenic aldehyde, 2-methoxy-4-propylphenol, 3-methyl-5-phenyl-2-pentenenitrile, 1-(spiro[4.5]dec-6/7-en-7-yl)-4-penten-1-one(, 2-methoxynaphthalene, (-)-(3aR,5AS,9AS,9BR)-3a,6,6,9a-tetramethyldodecahydronaphtho[2,1-b]furan, 5-nonanolide, (3aR,5AS,9AS,9BR)-3a,6,6,9a-tetramethyldodecahydronaphtho[2,1-b]furan, 7-isopropyl-2H,4H-1,5-benzodioxepin-3-one, coumarin, 4-methylphenyl isobutyrate, (2E)-1-(2,6,6-trimethyl-1,3-cyclohexadien-1-yl)-2-buten-1-one, beta,2,2,3-tetramethyl-delta-methylene-3-cyclopentene-1-butanol, delta damascone ((2E)-1-[(1RS,2SR)-2,6,6-trimethyl-3-cyclohexen-1-yl]-2-buten-1-one), (+-)-3,6-dihydro-4,6-dimethyl-2-phenyl-2h-pyran, anisaldehyde, paracresol, 3-ethoxy-4-hydroxybenzaldehyde, methyl 2-aminobenzoate, ethyl methylphenylglycidate, octalactone gamma, ethyl 3-phenyl-2-propenoate, (-)-(2E)-2-ethyl-4-[(1R)-2,2,3-trimethyl-3-cyclopenten-1-yl]-2-buten-1-ol, paracresyl acetate, dodecalactone, tricyclone, (+)-(3R,5Z)-3-methyl-5-cyclopentadecen-1-one, undecalactone, (1R,4R)-8-mercapto-3-p-menthanone, (3S,3AS,6R,7AR)-3,6-dimethylhexahydro-1-benzofuran-2(3H)-one, beta ionone, (+-)-6-pentyltetrahydro-2H-pyran-2-one, (3E,5Z)-1,3,5-undecatriene, 10-undecenal, (9E)-9-undecenal (9Z)-9-undecenal, (Z)-4-decenal, (+-)-ethyl 2-methylpentanoate, 1,2-diallyldisulfane, 2-tridecenenitrile, 3-tridecenenitrile, , (+-)-2-ethyl-4,4-dimethyl-1,3-oxathiane, (+)-(3R,5Z)-3-methyl-5-cyclopentadecen-1-one, 3-(4-tert-butylphenyl)propanal, allyl (cyclohexyloxy)acetate, methylnaphthylketone, (+-)-(4E)-3-methyl-4-cyclopentadecen-1-one, (+-)-5E3-methyl-5-cyclopentadecen-1-one, cyclopropylmethyl 3-hexenoate, (4E)-4-methyl-5-(4-methylphenyl)-4-pentenal, (+-)-1-(5-propyl-1,3-benzodioxol-2-yl)ethanone, 4-methyl-2-pentylpyridine, (+-)-(E)-3-methyl-4-(2,6,6-trimethyl-2-cyclohexen-1-yl)-3-buten-2-one, (3aRS,5aSR,9aSR,9bRS)-3a,6,6,9a-tetramethyldodecahydronaphtho[2,1-b]furan, (2S,5R)-5-methyl-2-(2-propanyl)cyclohexanone oxime, 6-hexyltetrahydro-2H-pyran-2-one, (+-)-3-(3-isopropyl-1-phenyl)butanal, methyl 2-(3-oxo-2-pentylcyclopentyl)acetate, 1-(2,6,6-trimethyl-1-cyclohex-2-enyl)pent-1-en-3-one, indol, 7-propyl-2H,4H-1,5-benzodioxepin-3-one, ethyl praline, (4-methylphenoxy)acetaldehyde, ethyl tricyclo[5.2.1. 0.^{2,6}]decane-2-carboxylate, (+)-(1'S,2S,E)-3,3-dimethyl-5-(2',2',3'-trimethyl-3'-cyclopenten-1'-yl)-4-penten-2-ol, (4E)-3,3-dimethyl-5-[(1R)-2,2,3-trimethyl-3-cyclopenten-1-yl]-4-penten-2-ol, 8-isopropyl-6-methyl-bicyclo[2.2.2]oct-5-ene-2-carbaldehyde, methylnonylacetaldehyde, 4-formyl-2-methoxyphenyl 2-methylpropanoate, (E)-4-decenal, (+-)-2-ethyl-4-(2,2,3-trimethyl-3-cyclopenten-1-yl)-2-buten-1-ol, (1R,5R)-4,7,7-trimethyl-6-thiabicyclo[3.2.1]oct-3-ene, (1R,4R,5R)-4,7,7-trimethyl-6-thiabicyclo[3.2.1]octane, (-)-(3R)-3,7-dimethyl-1,6-octadien-3-ol, (E)-3-phenyl-2-propenenitrile, 4-methoxybenzyl acetate, (E)-3-methyl-5-(2,2,3-trimethyl-3-cyclopenten-1-yl)-4-penten-2-ol, allyl (2/3-methylbutoxy)acetate, (+-)-(2E)-1-(2,6,6-trimethyl-2-cyclohexen-1-yl)-2-buten-1-one, (1E)-1-(2,6,6-trimethyl-1-cyclohexen-1-yl)-1-penten-3-one, and mixtures thereof.

According to an embodiment, perfume raw materials having a Log T<-4 are chosen in the group consisting of aldehydes, ketones, alcohols, phenols, esters lactones, ethers, epoxides, nitriles and mixtures thereof.

According to an embodiment, perfume raw materials having a Log T<-4 comprise at least one compound chosen in the group consisting of alcohols, phenols, esters lactones, ethers, epoxydes, nitriles and mixtures thereof, preferably in amount comprised between 20 and 70% by weight based on the total weight of the perfume raw materials having a Log T<-4.

According to an embodiment, perfume raw materials having a Log T<-4 comprise between 20 and 70% by weight of aldehydes, ketones, and mixtures thereof based on the total weight of the perfume raw materials having a Log T<-4.

The remaining perfume raw materials contained in the oil-based core may have therefore a Log T>-4.

According to an embodiment, the perfume raw materials having a Log T>-4 are chosen in the group consisting of ethyl 2-methylbutyrate, (E)-3-phenyl-2-propenyl acetate, (+-)-6/8-sec-butylquinoline, (+-)-3-(1,3-benzodioxol-5-yl)-2-methylpropanal, verdyl propionate, 1-(octahydro-2,3,8,8-tetramethyl-2-naphtalenyl)-1-ethanone, methyl 2-((1RS,2RS)-3-oxo-2-pentylcyclopentyl)acetate, (+-)-(E)-4-methyl-3-decen-5-ol, 2,4-dimethyl-3-cyclohexene-1-carbaldehyde, 1,3,3-trimethyl-2-oxabicyclo[2.2.2]octane, tetrahydro-4-methyl-2-(2-methyl-1-propenyl)-2H-pyran, dodecanal, 1-oxa-12/13-cyclohexadecen-2-one, (+-)-3-(4-isopropylphenyl)-2-methylpropanal, aldehyde C11, (+-)-2,6-dimethyl-7-octen-2-ol, allyl 3-cyclohexylpropanoate, (Z)-3-hexenyl acetate, 5-methyl-2-(2-propanyl)cyclohexanone, allyl heptanoate, 2-(2-methyl-2-propanyl)cyclohexyl acetate, 1,1-dimethyl-2-phenylethyl butyrate, geranyl acetate, neryl acetate, (+-)-1-phenylethyl acetate, 1,1-dimethyl-2-phenylethyl acetate, 3-methyl-2-butenyl acetate, ethyl 3-oxobutanoate, (2Z)-ethyl 3-hydroxy-2-butenoate, 8-p-menthanol, 8-p-menthanyl acetate, 1-p-menthanyl acetate, (+-)-2-(4-methyl-3-cyclohexen-1-yl)-2-propanyl acetate, (+-)-2-methylbutyl butanoate, 2-{(1S)-1-[(1R)-3,3-dimethylcyclohexyl]ethoxy}-2-oxoethyl propionate, 3,5,6-trimethyl-3-cyclohexene-1-carbaldehyde, 2,4,6-trimethyl-3-cyclohexene-1-carbaldehyde, 2-cyclohexylethyl acetate, octanal, ethyl butanoate, (+-)-(3E)-4-(2,6,6-trimethyl-1/2-cyclohexen-1-yl)-3-buten-2-one, 1-[(1RS,6SR)-2,2,6-trimethylcyclohexyl]-3-hexanol, 1,3,3-trimethyl-2-oxabicyclo[2.2.2]octane, 1,3,3-trimethyl-2-oxabicyclo[2.2.2]octane, ethyl hexanoate, undecanal, decanal, 2-phenylethyl acetate, (1S,2S,4S)-1,7,7-trimethylbicyclo[2.2.1]heptan-2-ol, (1S,2R,4S)-1,7,7-trimethylbicyclo[2.2.1]heptan-2-ol ), (+-)-3,7-dimethyl-3-octanol, 1-methyl-4-(2-propanylidene)cyclohexene, (+)-(R)-4-(2-methoxypropan-2-yl)-1-methylcyclohex-1-ene, verdyl acetate, (3R)-1-[(1R,6S)-2,2,6-trimethylcyclohexyl]-3-hexanol, (3S)-1-[(1R,65)-2,2,6-trimethylcyclohexyl]-3-hexanol, (3R)-1-[(1S,6S)-2,2,6-trimethylcyclohexyl]-3-hexanol, (+)-(1S,1'R)-2-[1-(3',3'-dimethyl-1'-cyclohexyl)ethoxy]-2-methyl propyl propanoate, and mixtures thereof.

According to an embodiment, the perfume formulation comprises
- 0 to 60 wt.% of a hydrophobic solvent (based on the total weight of the perfume formulation),
- 40 to 100 wt.% of a perfume oil (based on the total weight of the perfume formulation), wherein the perfume oil has at least two, preferably all of the following characteristics:
   ∘ at least 35%, preferably at least 40%, preferably at least 50%, more preferably at least 60% of perfuming ingredients having a log P above 3, preferably above 3.5,
   ∘ at least 20%, preferably at least 25%, preferably at least 30%, more preferably at least 40% of Bulky materials of groups 1 to 6, preferably 3 to 6 as previously defined and
   ∘ at least 15%, preferably at least 20%, more preferably at least 25%, even more preferably at least 30% of high impact perfume materials having a Log T < -4 as previously defined,
   optionally, further hydrophobic active ingredients.

According to a particular embodiment, the perfume comprises 0 to 60 wt.% of a hydrophobic solvent.

According to a particular embodiment, the hydrophobic solvent is a density balancing material preferably chosen in the group consisting of benzyl salicylate, benzyl benzoate, cyclohexyl salicylate, benzyl phenylacetate, phenylethyl phenylacetate, triacetin, ethyl citrate, methyl and ethyl salicylate, benzyl cinnamate, and mixtures thereof.

In a particular embodiment, the hydrophobic solvent has Hansen Solubility Parameters compatible with entrapped perfume oil.

The term "Hansen solubility parameter" is understood refers to a solubility parameter approach proposed by Charles Hansen used to predict polymer solubility and was developed around the basis that the total energy of vaporization of a liquid consists of several individual parts. To calculate the "weighted Hansen solubility parameter" one must combine the effects of (atomic) dispersion forces, (molecular) permanent dipole-permanent dipole forces, and (molecular) hydrogen bonding (electron exchange). The weighted Hansen solubility parameter" is calculated as (δD²+ δP²+ δH²)^{0.5}, wherein δD is the Hansen dispersion value (also referred to in the following as the atomic dispersion fore), δP is the Hansen polarizability value (also referred to in the following as the dipole moment), and δH is the Hansen Hydrogen-bonding ("h-bonding") value (also referred to in the following as hydrogen bonding). For a more detailed description of the parameters and values, see Charles Hansen, The Three Dimensional Solubility Parameter and Solvent Diffusion Coefficient, Danish Technical Press (Copenhagen, 1967). Euclidean difference in solubility parameter between a fragrance and a solvent is calculated as (4*(δDₛₒₗᵥₑₙₜ-δD_{fragrance})² + (δPₛₒₗᵥₑₙₜ-δP_{fragrance})² + (δHₛₒₗᵥₑₙₜ-δH_{fragrance})²)^{0.5}, in which δDₛₒₗᵥₑₙₜ, δPₛₒₗᵥₑₙₜ, and δHₛₒₗᵥₑₙₜ, are the Hansen dispersion value, Hansen polarizability value, and Hansen h-bonding values of the solvent, respectively; and 8D_{fragrance}, 8P_{fragrance}, and 8H_{fragrance} are the Hansen dispersion value, Hansen polarizability value, and Hansen h-bonding values of the fragrance, respectively.

In a particular embodiment, the perfume oil and the hydrophobic solvent have at least two Hansen solubility parameters selected from a first group consisting of: an atomic dispersion force (δD) from 12 to 20, a dipole moment (δP) from 1 to 8, and a hydrogen bonding (δH) from 2.5 to 11.

In a particular embodiment, the perfume oil and the hydrophobic solvent have at least two Hansen solubility parameters selected from a second group consisting of: an atomic dispersion force (δD) from 12 to 20, preferably from 14 to 20, a dipole moment (δP) from 1 to 8, preferably from 1 to 7, and a hydrogen bonding (δH) from 2.5 to 11, preferably from 4 to 11.

In a particular embodiment, at least 90% of the perfume oil, preferably at least 95% of the perfume oil, most preferably at least of 98% of the perfume oil has at least two Hansen solubility parameters selected from a first group consisting of: an atomic dispersion force (δD) from 12 to 20, a dipole moment (δP) from 1 to 8, and a hydrogen bonding (δH) from 2.5 to 11.

In a particular embodiment, the perfume oil and the hydrophobic solvent have at least two Hansen solubility parameters selected from a second group consisting of: an atomic dispersion force (δD) from 12 to 20, preferably from 14 to 20, a dipole moment (δP) from 1 to 8, preferably from 1 to 7, and a hydrogen bonding (δH) from 2.5 to 11, preferably from 4 to 11.

According to an embodiment, the perfuming formulation comprises a fragrance modulator (that can be used in addition to the hydrophobic solvent when present or as substitution of the hydrophobic solvent when there is no hydrophobic solvent).

Preferably, the fragrance modulator is defined as a fragrance material with
i. a vapor pressure of less than 0.0008 Torr at 22°C;
ii. a clogP of 3.5 and higher, preferably 4.0 and higher and more preferably 4.5
iii. at least two Hansen solubility parameters selected from a first group consisting of: an atomic dispersion force from 12 to 20, a dipole moment from 1 to 7, and a hydrogen bonding from 2.5 to 11,
iv. at least two Hansen solubility parameters selected from a second group consisting of: an atomic dispersion force from 14 to 20, a dipole moment from 1 to 8, and a hydrogen bonding from 4 to 11, when in solution with a compound having a vapor pressure range of 0.0008 to 0.08 Torr at 22°C.

Preferably, as examples the following ingredients can be listed as modulators but the list in not limited to the following materials: alcohol C12, oxacyclohexadec-12/13-en-2-one, 3-[(2',2',3'-trimethyl-3'-cyclopenten-1'-yl)methoxy]-2-butanol, cyclohexadecanone, (Z)-4-cyclopentadecen-1-one, cyclopentadecanone, (8Z)-oxacycloheptadec-8-en-2-one, 2-[5-(tetrahydro-5-methyl-5-vinyl-2-furyl)-tetrahydro-5-methyl-2-furyl]-2-propanol, muguet aldehyde, 1,5,8-trimethyl-13-oxabicyclo[10.1.0]trideca-4,8-diene, (+-)-4,6,6,7,8,8-hexamethyl-1,3,4,6,7,8-hexahydrocyclopenta[g]isochromene, (+)-(1S,2S,3S,5R)-2,6,6-trimethylspiro[bicyclo[3.1.1]heptane-3,1'-cyclohexane]-2'-en-4'-one, oxacyclohexadecan-2-one, 2-{(1S)-1-[(1R)-3,3-dimethylcyclohexyl]ethoxy}-2-oxoethyl propionate, (+)-(4R,4aS,6R)-4,4a-dimethyl-6-(1-propen-2-yl)-4,4a,5,6,7,8-hexahydro-2(3H)-naphthalenone, amylcinnamic aldehyde, hexylcinnamic aldehyde, hexyl salicylate, (1E)-1-(2,6,6-trimethyl-1-cyclohexen-1-yl)-1,6-heptadien-3-one, (9Z)-9-cycloheptadecen-1-one.

According to a particular embodiment, the hydrophobic material is free of any active ingredient (such as perfume). According to this particular embodiment, it comprises, preferably consists of hydrophobic solvents, preferably chosen in the group consisting of isopropyl myristate, tryglycerides (e.g. Neobee^{®} MCT oil, vegetable oils), D-limonene, silicone oil, mineral oil, and mixtures thereof with optionally hydrophilic solvents preferably chosen in the group consisting of 1,4-butanediol, benzyl alcohol, triethyl citrate, triacetin, benzyl acetate, ethyl acetate, propylene glycol (1,2-propanediol), 1,3-propanediol, dipropylene glycol, glycerol, glycol ethers and mixtures thereof .

The term "biocide" refers to a chemical substance capable of killing living organisms (e.g. microorganisms) or reducing or preventing their growth and/or accumulation. Biocides are commonly used in medicine, agriculture, forestry, and in industry where they prevent the fouling of, for example, water, agricultural products including seed, and oil pipelines. A biocide can be a pesticide, including a fungicide, herbicide, insecticide, algicide, molluscicide, miticide and rodenticide; and/or an antimicrobial such as a germicide, antibiotic, antibacterial, antiviral, antifungal, antiprotozoal and/or antiparasite.

As used herein, a "pest control agent" indicates a substance that serves to repel or attract pests, to decrease, inhibit or promote their growth, development or their activity. Pests refer to any living organism, whether animal, plant or fungus, which is invasive or troublesome to plants or animals, pests include insects notably arthropods, mites, spiders, fungi, weeds, bacteria and other microorganisms.

According an embodiment, the hydrophobic material represents between about 10% and 95% by weight, relative to the total weight of the oil phase. According another embodiment, the hydrophobic material represents between about 10% and 80% by weight, relative to the total weight of the oil phase. According another embodiment, the hydrophobic material represents between about 10% and 60% by weight, relative to the total weight of the oil phase. According another embodiment, the hydrophobic material represents between about 15% and 45% by weight, relative to the total weight of the oil phase.

According to a particular embodiment, the oil phase essentially consists of the polyfunctional monomer and a perfume or flavor oil.

According to an embodiment, a first type of catalyst can be added during the process to initiate the polymerization of polyfunctional monomers (particularly when (meth)acrylate monomers are used as functional monomers) and/or a second type of catalyst can be added during the process to enhance the reaction between acetoacetate groups of the acetoacetate polymer with polyfunctional monomers (such as for example amines, (meth)acrylate monomers).

Catalyst(s) can be added in the oil phase (step 2) and/or in the emulsion (step 3).

The first type of catalyst are free radical initiators, including for example organic peroxides, such as Benzoyl peroxide, Dicumyl peroxide, Di-tert-butyl peroxide, 2,5-Bis(tert-butylperoxy)-2,5-dimethylhexane, Cumene hydroperoxide, tert-Butyl hydroperoxide, 2,4-Pentanedione peroxide, 2-Butanone peroxide, Lauroyl peroxide, tert-Butyl peroxybenzoate, 2,5-Di(tert-butylperoxy)-2,5-dimethyl-3-hexyne, tert-Butyl peracetate, 1,1-Bis(tert-butylperoxy)cyclohexane; or selecting from the group of Azo compounds, such as 2,2'-Azobis(2-methylpropionitrile), 2,2'-Azobis(2-methylbutyronitrile), 2,2'-Azobis(2,4-dimethyl)valeronitrile, 4,4'-Azobis(4-cyanovaleric acid), Dimethyl 2,2'-azobis(2-methylpropionate), 1,1'-Azobis(cyclohexanecarbonitrile), 2,2'-Azobis(2-methylpropionamidine) dihydrochloride, 2,2'-Azobis[2-(2-imidazolin-2-yl)propane] Dihydrochloride; or/and inorganic peroxides, such as Sodium persulfate, Potassium persulfate, Ammonium persulfate, Hydroxymethanesulfinic acid monosodium salt, Hydrogen peroxide, and mixtures thereof. In a particular embodiment, the free radical initiator is a redox system which can produce radical species under mild condition. Examples of the redox system include hydrogen peroxide and a metal salt (such as ferrous ion, cuprous ion, cobalt ion), an organic peroxide (such as Benzoyl peroxide, Di-tert-butyl peroxide) and a metal salt (such as cuprous ion, cobalt ion), an organic peroxide and a tertiary amine, an inorganic peroxide (such as Sodium persulfate, Potassium persulfate, Ammonium persulfate) and ferrous ion, an inorganic peroxide and a sulfite, an inorganic peroxide and a thiosulfate.

The second type of catalysts are base catalysts, such as alkali metal hydroxides, alkali metal alkoxides, metal carbonate, alkali and alkali-earth metal oxide-based catalysts; or/and nucleophilic catalysts selecting from the group of Lewis bases, such as primary amines, secondary amines, tertiary amines, pyridine-based catalysts (e.g., Pyridine, 4-dimethylaminopyridine, pyridonaphthyridine, 4-pyrrolidinopyridine (4-PPY)), amidine-based catalysts (e.g., 1,8-Diazabicyclo(5.4.0)undec-7-ene (DBU), 1,5-Diazabicyclo(4.3.0)non-5-ene (DBN)), imidazole, phosphine-based catalysts (e.g., Tri-n-butylphosphine, Tri-tert-butylphosphine) and mixtures thereof.

### Polymeric shell

According to an embodiment, the polymeric shell comprises a polymeric material.

According to an embodiment, the polymeric shell comprises (or is made of) a material selected from the group consisting of polyenamine, polyurea, polyurethane, polyamide, polyester, polyacrylate, polysiloxane, polycarbonate, polysulfonamide, polymers of urea and formaldehyde, melamine and formaldehyde, melamine and urea, melamine and glyoxal, gelatin/gum arabic and mixtures thereof.

According to a particular embodiment, the polymeric material is polyenamine.

According to a particular embodiment, the polymeric material is poly(meth)acrylate.

According to a particular embodiment, the polymeric material is polyurea and/or polyurethane.

According to a particular embodiment, the polymeric material is polyamide.

According to an embodiment, the polymeric material is present in an amount less than 30% by weight based on the total weight of the microcapsule.

According to another embodiment, the polymeric material is present in an amount less than 20% by weight based on the total weight of the microcapsule.

According to another embodiment, the polymeric material is present in an amount less than 10% by weight based on the total weight of the microcapsule.

According to an embodiment, the polymeric material is present in an amount less than 12% by weight based on the total weight of the microcapsule slurry.

According to another embodiment, the polymeric material is present in an amount less than 8% by weight based on the total weight of the microcapsule slurry.

According to another embodiment, the polymeric material is present in an amount less than 5% by weight based on the total weight of the microcapsule slurry.

Indeed, it has been underlined that even with a reduced amount of the polymeric material forming the shell, microcapsules still show good stability in consumer products.

### Acetoacetyl polymer

According to an embodiment, the acetoacetyl polymer is in the form of solid particles.

According to an embodiment, the acetoacetyl polymer is used as a stabilizer and can be embedded within the shell.

According to a particular embodiment, the acetoacetyl polymer acts as reactive stabilizer and is embedded within the shell.

By reactive stabilizer, it means that the stabilizer acts both as a stabilizer to stabilize the oil-in-water emulsion and as reactant that reacts with the polyfunctional monomer.

Indeed, depending on the nature of the polyfunctional monomer, the acetoacetyl polymer can also react with said polyfunctional monomer to form the polymerized shell.

According to a particular embodiment, the acetoacetyl polymer reacts with the polyfunctional monomer to form the polymerized shell.

Preferred acetoacetyl polymer suspension (in water) is those having polymer content between 0.01% to 10%, more preferably between 0.01% to 5.0% by weight based on the total weight of the suspension.

Acetoacetyl polymer is comprised within the polymeric shell, meaning that it preferably participates to the polymeric shell formation and has covalent bond interactions with the polymeric shell, or incorporates into the polymeric shell or/and adheres to the polymeric shell under non-covalent interactions.

According to a particular embodiment, the acetoacetyl polymer is chosen from cellulose acetoacetate, starch acetoacetate, polyfunctional acetoacetate having two to six acetoacetate functional groups and mixtures thereof. Polyfunctional acetoacetate having two to six acetoacetate functional groups can be chosen in the group consisting of ethyl diacetoacetate, 1,3-butanediol diacetoacetate, ethylene diacetoacetate, titanium diisopropoxide bis(ethyl acetoacetate), ethyl 2,2'-(4-methoxybenzal)bis-acetoacetate, neopentylglycolycol bis acetoacetate, ethylene glycol diacetoacetate, trimethylolpropane triacetoacetate, pentaerythritol tetraacetoacetate and mixtures thereof.

The acetoacetyl polymer is not an acetoacetyl polyvinyl alcohol (PVOH) or an acetoacetyl modified polyvinyl alcohol (PVOH).

The acetoacetyl polymer can be prepared by using different methods well-known from the person skilled in the art.

According to an embodiment, the acetoacetyl polymer is obtained by the following process:
(i) dissolving a polymer having hydroxyl groups into a solvent to form a homogeneous solution
(ii) inducing acetoacetylation of said polymer via reaction with the hydroxyl groups
(iii) inducing precipitation of the polymer from the solution obtained in step (ii),
(iv) Optionally, purifying the precipitated polymer, preferably by a washing process to obtain an acetoacetyl polymer.

As an example, one may use ionic liquid as solvent in step (i) to dissolve cellulose or dimethyl sulfoxide as a solvent to dissolve starch.

Step ii) can comprise:
(1) reaction of corresponding hydroxy compounds with diketene or diketene derivatives; or
(2) transesterification of corresponding hydroxy compounds with acetoacetate (such as tert-butyl acetoacetate, methyl acetoacetate, isobutyl acetoacetate, 2-methoxyethyl acetoacetate, ethyl (4-methylbenzoyl) acetate, ethyl 3-oxohexanoate).

In step ii), the polymer can precipitate from the homogenous solution of step i) by adding an anti-solvent to the polymer or/and by removing the solvent in homogeneous solution.

Solvent used in step (i) is a solvent or a mixture which can dissolve the polymer to form homogeneous solution. The person skilled in the art will be able to select suitable solvent(s). It can be for example 1-allyl-3-methylimidazolium chloride (a type of ionic liquid) or Dimethylformamide (DMF) for cellulose and dimethyl sulfoxide for starch.

A heating step can be carried out in step (i).

The person skilled in the art will be able to select suitable anti-solvent(s). As anti-solvent, one may cite for example ethanol, acetone, ethyl acetate, water, acid solution, a salt solution and mixtures thereof.

In a particular embodiment, the shell material comprises a biodegradable material.

In a particular embodiment, the shell has a biodegradability of at least 40%, preferably at least 45%, 50%, 60%, 65%, 70%, 75%, 80%, 85%, 90%, 95% or 98%, within 60 days according to OECD301F.

In a particular embodiment, the core-shell microcapsule has a biodegradability of at least 40 %, preferably at least 60 %, preferably at least 65%, 70%, 75%, 80%, 85%, 90%, 95% or 98% within 60 days according to OECD301F.

Thereby it is understood that the core-shell microcapsule including all components, such as the core, shell and optionally coating can have a biodegradability of at least 40 %, preferably at least 60 %, preferably at least 65%, 70%, 75%, 80%, 85%, 90%, 95% or 98% within 60 days according to OECD301F.

In a particular embodiment, the oil-based core, preferably perfume oil has a biodegradability of at least 40 %, preferably at least 60 %, preferably at least 65%, 70%, 75%, 80%, 85%, 90%, 95% or 98% within 60 days according to OECD301F.

OECD301F is a standard test method on the biodegradability from the Organization of Economic Co-operation and Development.

A typical method for extracting the shell for measuring the biodegradability is disclosed in Gasparini and all in Molecules 2020, 25,718*.*

### Optional components

When microcapsules are in the form of a slurry, the microcapsule slurry can comprise auxiliary ingredients selected from the group of thickening agents/rheology modifiers, antimicrobial agents, opacity-building agents, mica particles, salt, pH stabilizers/buffering ingredients, preferably in an amount comprised between 0 and 15% by weight based on the total weight of the slurry.

According to another embodiment, the microcapsule slurry of the invention comprises additional free (i.e non-encapsulated) perfume, preferably in an amount comprised between 5 and 50% by weight based on the total weight of the slurry.

### Optional outer coating

According to a particular embodiment of the invention, microcapsules according to the invention comprise an outer coating material selected from the group consisting of a polysaccharide, a cationic polymer, a polysuccinimide derivative (as described for instance in WO2021185724) and mixtures thereof to form an outer coating to the microcapsule.

Polysaccharide polymers are well known to a person skilled in the art. Preferred non-ionic polysaccharides are selected from the group consisting of locust bean gum, xyloglucan, guar gum, hydroxypropyl guar, hydroxypropyl cellulose and hydroxypropyl methyl cellulose, pectin and mixtures thereof.

According to a particular embodiment, the coating consists of a cationic coating.

Cationic polymers are also well known to a person skilled in the art. Preferred cationic polymers have cationic charge densities of at least 0.5 meq/g, more preferably at least about 1.5 meq/g, but also preferably less than about 7 meq/g, more preferably less than about 6.2 meq/g. The cationic charge density of the cationic polymers may be determined by the Kjeldahl method as described in the US Pharmacopoeia under chemical tests for Nitrogen determination. The preferred cationic polymers are chosen from those that contain units comprising primary, secondary, tertiary and/or quaternary amine groups that can either form part of the main polymer chain or can be borne by a side substituent directly connected thereto. The weight average (Mw) molecular weight of the cationic polymer is preferably between 10,000 and 3.5M Dalton, more preferably between 50,000 and 2M Dalton.

According to a particular embodiment, one will use cationic polymers based on acrylamide, methacrylamide, N-vinylpyrrolidone, quaternized N,N-dimethylaminomethacrylate, diallyldimethylammonium chloride, quaternized vinylimidazole (3-methyl-1-vinyl-1H-imidazol-3-ium chloride), vinylpyrrolidone, acrylamidopropyltrimonium chloride, cassia hydroxypropyltrimonium chloride, guar hydroxypropyltrimonium chloride or polygalactomannan 2-hydroxypropyltrimethylammonium chloride ether, starch hydroxypropyltrimonium chloride and cellulose hydroxypropyltrimonium chloride. Preferably copolymers shall be selected from the group consisting of polyquaternium-5, polyquaternium-6, polyquaternium-7, polyquaternium10, polyquaternium-11, polyquaternium-16, polyquaternium-22, polyquaternium-28, polyquaternium-43, polyquaternium-44, polyquaternium-46, cassia hydroxypropyltrimonium chloride, guar hydroxypropyltrimonium chloride or polygalactomannan 2-hydroxypropyltrimethylammonium chloride ether, starch hydroxypropyltrimonium chloride and cellulose hydroxypropyltrimonium chloride

As specific examples of commercially available products, one may cite Salcare^{®} SC60 (cationic copolymer of acrylamidopropyltrimonium chloride and acrylamide, origin: BASF) or Luviquat^{®}, such as the PQ 11N, FC 550 or Style (polyquaternium-11 to 68 or quaternized copolymers of vinylpyrrolidone origin: BASF), or also the Jaguar^{®} (C13S or C17, origin Rhodia).

According to any one of the above embodiments of the invention, there is added an amount of polymer described above comprised between about 0% and 5% w/w, or even between about 0.1% and 2% w/w, percentage being expressed on a w/w basis relative to the total weight of the microcapsules or the slurry. It is clearly understood by a person skilled in the art that only part of said added polymers will be incorporated into/deposited on the microcapsule shell.

### PROCESS FOR PREPARING CORE-SHELL MICROCAPSULE

Core-shell microcapsules of the invention can be prepared according different processes depending notably on the nature of the polymeric wall.

Another object of the invention is a process for preparing core-shell microcapsules as defined above, wherein the process comprises the steps of:
1) preparing a water phase;
2) preparing an oil phase comprising a hydrophobic material, preferably a perfume oil;
3) adding the oil phase to the water phase and mixing them to form an oil-in-water emulsion, under conditions allowing the formation of core-shell microcapsule by interfacial polymerization and/or interfacial reaction,

wherein a polyfunctional monomer is added in step 1) in the water phase and/or in step 2) in the oil phase and/or in step 3) in the emulsion, and
wherein an acetoacetyl polymer is added in the water phase and/or in the oil phase or/and in the emulsion.

According to an embodiment, a polyfunctional monomer is added in step 1) in the water phase and/or in step 2) in the oil phase.

According to an embodiment, the process comprises the steps of:
1) preparing a water phase comprising an acetoacetyl polymer;
2) preparing an oil phase comprising a hydrophobic material, preferably a perfume oil;
3) adding the oil phase to the water phase and mixing them to form an oil-in-water emulsion, under conditions allowing the formation of core-shell microcapsule by interfacial polymerization and/or interfacial reaction,
wherein a polyfunctional monomer is added in step 1) in the water phase and/or in step 2) in the oil phase.

According to an embodiment, step 3) comprises:
- Step 3a) adding the oil phase into the water phase to form an oil-in-water emulsion, followed by
- Step 3b) applying conditions allowing the reaction (by interfacial polymerization and/or interfacial reaction) of the polyfunctional monomer to form polymeric shell at oil-water interface in the presence of an acetoacetyl polymer.

According to an embodiment, the acetoacetyl polymer in step 1) are in the form of a solution or a suspension.

According to a particular embodiment, the interfacial polymerization and/or interfacial reaction takes place between the polyfunctional monomer and the acetoacetyl polymer. According to this embodiment, the shell is formed via the reaction between the polyfunctional monomer (for example in oil), with acetoacetyl polymer, water and/or additional reactants in water phase at oil-in-water interface.

According to an embodiment, the polyfunctional monomer is added in the oil phase in step 2).

The previous embodiment is particularly suitable, when the polyfunctional monomer is soluble in oil (for example when polyisocyanate or oil soluble polyamine is used as a polyfunctional monomer).

According to an embodiment, the polyfunctional monomer is added in the water phase in step 1).

The previous embodiment is particularly suitable, when the polyfunctional monomer is soluble in water (for example when a melamine-based resin or a water-soluble polyamine is used as a polyfunctional monomer).

According to an embodiment, the polyfunctional monomer is added in the emulsion in step 3).

According to an embodiment, a first polyfunctional monomer is added in the water phase in step 1) (for example a melamine-based resin or a water-soluble polyamine) and a second polyfunctional monomer (for example a polyisocyanate or an oil soluble polyamine) is added in the oil phase in step 2).

According to an embodiment, a first polyfunctional monomer is added in the oil phase in step 1) and a second polyfunctional monomer is added in the emulsion in step 3).

According to a particular embodiment, the process of the invention comprises the step of adding an additional polymeric emulsifier (beyond the acetoacetyl polymer) in step 1) in the water phase.

By "polymeric emulsifier", it meant an emulsifier having both a polar group with an affinity for water (hydrophilic) and a nonpolar group with an affinity for oil (lipophilic). The hydrophilic part will dissolve in the water phase and the hydrophobic part will dissolve in the oil phase providing a film around droplets.

This optional polymeric emulsifier can allow assisting to stabilize the oil droplets in the presence of acetoacetyl polymer. This embodiment can be particularly suitable when acetoacetyl polymer concentration is low. The polymeric emulsifier can be an ionic or non-ionic surfactant. As non-limiting examples, non-ionic polymers include polyvinyl alcohol, gum Arabic, cellulose derivatives such hydroxyethyl cellulose, polyethylene oxide, co-polymers of polyethylene oxide and polyethylene or polypropylene oxide, co-polymers alkyl acrylates and N-vinypyrrolidone, and non-ionic polysaccharide. Ionic polymers include co-polymers of acrylamide and acrylic acid, acid anionic surfactant (such as sodium dodecyl sulfate), acrylic co-polymers bearing a sulfonate group, and co-polymers of vinyl ethers and maleic anhydride, and ionic polysaccharide.

According to an embodiment, during the process, no polymeric emulsifier is added at any stage of the process.

According to a particular embodiment, the process of the invention comprises the step of adding a colloidal stabilizer or colloidal particle stabilizer (in addition to the acetoacetyl polymer), in step 1), in the water phase.

According to a particular embodiment, the process of the invention comprises the step of adding a reactant in step 1) and/or step 3). This optional reactant can participate to the shell formation of microcapsules. The reactant can be water soluble or water suspensible. Examples of suitable reactants include amines, alcohols, phenols, thiols, and mixtures thereof.

The reactant is usually added in an amount comprised between 0.01% and 10%, preferably 0.01%and 5%, based on the total weight of the water phase.

When a reactant is added, the reactant can also react with the polyfunctional monomer for the polymeric shell formation. According to this embodiment, in addition to the reactant, the acetoacetyl polymer can also participate to the polymeric shell formation.

According to an embodiment, during the process, no reactant is added at any stage of the process.

In the first step of the process, the acetoacetyl polymer is dispersed in an aqueous phase. Typically, this is done using high mechanical agitation.

Said acetoacetyl polymer suspension may be obtained by the method described previously.

According to an embodiment, the total amount of acetoacetyl polymer present in water phase is comprised between 0.01 and 10 wt%, preferably between 0.01 and 5 wt% based on the total weight of the water phase.

According to an embodiment, the total amount of acetoacetyl polymer present in water phase is comprised between 0.01 and 10 wt%, preferably between 0.01 and 5 wt% based on the total weight of the emulsion.

According to an embodiment, in a second step, at least one oil soluble polyfunctional monomer is dissolved in a hydrophobic material (for example, a perfume or flavour oil) to form an oil phase, which is then added to the water phase to form a emulsion, the mean droplet size of which is comprised between 1 and 3000 microns, preferably between 1 and 500 microns, more preferably between 5 and 50 microns. The oil-in-water emulsion is made for instance by using high speed mechanical disperser or ultrasonic dispersers at room temperature.

According to an embodiment, the emulsion formation takes place at room temperature. By "room temperature", it is meant typically a temperature comprised between 20 and 25°C. According to another embodiment, the emulsion formation takes place at a temperature below room temperature.

Once the emulsion is formed, the pH value is preferably maintained at 4-9, or adjusted to a value above 6.5, or adjusted to a value above 8.5 and preferably not higher than 11. However, this step can be omitted.

According to an embodiment, the oil phase represents between 5 and 60%, preferably between 20 and 40% by weight of the emulsion.

Then, the interfacial polymerization and/or interfacial reaction can be carried out typically at a temperature between 25°C and 90°C, preferably between 40°C and 80°C under stirring for 2 to 40 hours to complete the reaction and form microcapsules in the form of a slurry. However, the heating step can be omitted.

According to an embodiment, the interfacial polymerization/reaction was carried out in the protection of inert gas (such as nitrogen), especially when (meth) acrylates are used as polyfunctional monomers.

According to an embodiment, the polyfunctional monomer is chosen in the group consisting of at least one polyamine, polyisocyanate, poly anhydride, poly maleic anhydride, poly acid chloride (acyl chloride), polyepoxide, acrylate monomers or (meth)acrylate monomers, polyalkoxysilane or alkoxysilane (such as vinyltriethoxysilane or (3-Aminopropyl) triethoxysilane), melamine-based resin, and mixtures thereof.
"Poly acid chloride" and "acyl chloride" are used indifferently in the present invention.

Suitable polyamines are selected from the group consisting at least two amino groups. The polyamines may include ethylenediamine, 1 2-diaminopropane, 1,3-diaminopropane, 1,4-diaminobutane, 1,5-diaminopentane, 1,7-diaminoheptane, 1,8-diaminooctane, 1,9-diaminononane, 1,10-diaminodecane, 1,12-diaminododecane, hexamethylenediamine, phenylenediamine, diaminotoluene, 4-aminobenzylamine, xylylenediamine, triethylenetetramine, diethylenetriamine, spermidine, spermine, agmatine, tris(2-aminoethyl)amine, guanidine carbonate, 3,5-diamino-1,2,4triazole, 2,2,4(2,4,4)-trimethyl-1,6-hexanediamine, 1,3-cyclohexanebis(methylamine), N,N'-bis(3-aminopropyl)ethylenediamine, 1,3-diamino-2-hydroxypropane, 2,2'(ethylenedioxy)diethylamine, aminoguanidine bicarbonate, biguanide, cystamine, 1,1,1-tris(aminomethyl)ethane, polyethylenimine, polyetheramines, polyvinylamine, amino acid (e.g., lysine, cystine, glutamine, arginine), chitosan, protein (e.g., whey protein, caseinate, silk fibroin).

Suitable acrylate monomers or (meth) acrylate monomers can be selected from the group consisting at least two (meth)acrylate functionalities. The acrylate monomer or (meth) acrylate monomers may include pentaerythritol tri(meth)acrylate, trimethylolpropane tri(meth)acrylate, pentaerytrithol tetra(meth)acrylate, Tetra(ethylene glycol) di(meth)acrylate, dipentaerytrhitol penta(meth)acryalate, dipentaerytrithol hexa(meth)acrylate, tricyclodecane dimenthanol di(meth)acrylate, ethylene glycol di(meth)acrylate, di(ethylene glycol) di(meth)acrylate, triethylene glycol di(meth)acrylate, propylene glycol di(meth)acrylate, 1,4-butanediol di(meth)acrylate, 1,6-hexanediol di(meth)acrylate, glycerol di(meth)acrylate, 1,3-butanediol di(meth)acrylate, neopentyl glycol di(meth)acrylate, 1,10-decanediol di(meth)acrylate, pentaerythritol tri(meth)acrylate, pentaerythritol tetra(meth)acrylate, dipentaerythritol hexa(meth)acrylate, triallylformal tri(meth)acrylate, allyl methacrylate, trimethylol propane tri(meth)acrylate, tributanediol di(meth)acrylate, PEG 200 di(meth)acrylate, PEG 400 di(meth)acrylate, PEG 600 di(meth)acrylate, pentaerythritol-tetraacrylate, pentaerythritol triacrylate (PETIA), 1,4-butanediol diacrylate (BDA-2), ethylene glycol dimethacrylate, trimethylolpropane triacrylate, hexane diol diacrylate, ((2,4,6-trioxocyclohexane-1,3,5-triyl)tris(oxy))tris(ethane-2,1-diyl) triacrylate, tris(2-acryloyloxyethyl) isocyanurate, 1,3,5-triacryloylhexahydro-1.3.5-triazine, and mixtures thereof.

Suitable polyisocyanates used according to the invention include aromatic polyisocyanate, aliphatic polyisocyanate and mixtures thereof. Said polyisocyanate comprises at least 2, preferably at least 3 but may comprise up to 6, or even only 4, isocyanate functional groups. According to a particular embodiment, a diisocyanate (2 isocyanate functional group) or a triisocyanate (3 isocyanate functional group) or mixtures thereof is used.

According to one embodiment, said polyisocyanate is an aromatic polyisocyanate. The term "aromatic polyisocyanate" is meant here as encompassing any polyisocyanate comprising an aromatic moiety. Preferably, it comprises a phenyl, a toluyl, a xylyl, a naphthyl or a diphenyl moiety, more preferably a toluyl or a xylyl moiety. Preferred aromatic polyisocyanates are biurets, polyisocyanurates and trimethylol propane adducts of diisocyanates, more preferably comprising one of the above-cited specific aromatic moieties. More preferably, the aromatic polyisocyanate is a polyisocyanurate of toluene diisocyanate (commercially available from Bayer under the tradename Desmodur^{®} RC), a trimethylol propane-adduct of toluene diisocyanate (commercially available from Bayer under the tradename Desmodur^{®} L75), a trimethylol propane-adduct of xylylene diisocyanate (commercially available from Mitsui Chemicals under the tradename Takenate^{®} D-110N).

According to another embodiment, said polyisocyanate is an aliphatic polyisocyanate. The term "aliphatic polyisocyanate" is defined as a polyisocyanate which does not comprise any aromatic moiety. Preferred aliphatic polyisocyanates are a trimer of hexamethylene diisocyanate, a trimer of isophorone diisocyanate, a trimethylol propane-adduct of hexamethylene diisocyanate (available from Mitsui Chemicals), a biuret of hexamethylene diisocyanate (commercially available from Bayer under the tradename Desmodur^{®} N 100).

According to another embodiment, the polyisocyanate is in the form of a mixture of at least one aliphatic polyisocyanate and of at least one aromatic polyisocyanate, both comprising at least two or three isocyanate functional groups, such as a mixture of a biuret of hexamethylene diisocyanate with a trimethylol propane-adduct of xylylene diisocyanate, a mixture of a biuret of hexamethylene diisocyanate with a polyisocyanurate of toluene diisocyanate, and a mixture of a biuret of hexamethylene diisocyanate with a trimethylol propane-adduct of toluene diisocyanate. Most preferably, it is a mixture of a biuret of hexamethylene diisocyanate with a trimethylol propane-adduct of xylylene diisocyanate. Preferably, when used as a mixture the molar ratio between the aliphatic polyisocyanate and the aromatic polyisocyanate is ranging from 90:10 to 10:90.

According to an embodiment, the polyfunctional monomer used in the process of the invention is present in amounts representing from 0.1 and 30%, preferably from 0.2 and 20%, more preferably from 0.2 and 10% by weight based on the total amount of the oil phase.

According to an embodiment, the polyfunctional monomer used in the process of the invention is present in amounts representing from 0.1 and 30%, preferably from 0.2 and 20%, more preferably from 0.2 and 10% by weight based on the total amount of the microcapsule slurry.

### Optional step: optional outer coating

According to a particular embodiment of the invention, at the end of step 3) or during step 3), one may also add to the invention's slurry a polymer selected from the group consisting of a non-ionic polysaccharide, a cationic polymer a polysuccinimide derivative and mixtures thereof as defined previously to form an outer coating to the microcapsule. Non-ionic polysaccharide and cationic polymer are defined as previously.

### PROCESS FOR PREPARING MICROCAPSULE POWDER

Also disclosed is a process for preparing a microcapsule powder comprising the steps as defined above and an additional step consisting of submitting the slurry obtained in step 3) to a drying process, like spray-drying, to provide the microcapsules as such, i.e. in a powdery form. It is understood that any standard method known by a person skilled in the art to perform such drying is also applicable. In particular the slurry may be spray-dried preferably in the presence of a polymeric carrier material such as polyvinyl acetate, polyvinyl alcohol, dextrins, natural or modified starch, vegetable gums, pectins, xanthans, alginates, carragenans or cellulose derivatives to provide microcapsules in a powder form.

However, one may cite also other drying method such as the extrusion, plating, spray granulation, the fluidized bed, or even a drying at room temperature using materials (carrier, desiccant) that meet specific criteria as disclosed in WO2017/134179.

According to a particular embodiment, the carrier material contains free perfume oil which can be the same or different from the perfume from the core of the microcapsules.

### CORE-SHELL MICROCAPSULE

Also disclosed is a microcapsule or a microcapsule slurry obtainable by the process as described above.

### MULTIPLE CAPSULE SYSTEM

According to an embodiment, the microcapsules of the invention (first type of microcapsule) can be used in combination with a second type of microcapsules.

Also disclosed is a microcapsule delivery system comprising:
- the microcapsules of the present invention as a first type of microcapsules, and
- a second type of microcapsules, wherein the first type of microcapsules and the second type of microcapsules differ in their hydrophobic material and/or their wall material and/or in their coating material.

### PERFUMING COMPOSITION AND CONSUMER PRODUCTS

The microcapsules of the invention can be used in combination with active ingredients. Also disclosed is therefore a composition comprising:
(i) microcapsules or microcapsule slurry as defined above;
(ii) an active ingredient, preferably chosen in the group consisting of a cosmetic ingredient, skin caring ingredient, perfume ingredient, flavor ingredient, malodour counteracting ingredient, bactericide ingredient, fungicide ingredient, pharmaceutical or agrochemical ingredient, a sanitizing ingredient, an insect repellent or attractant, and mixtures thereof.

The microcapsules of the invention show a good performance in terms of stability in challenging medium.

Also disclosed is a perfuming composition comprising:
(i) microcapsules or microcapsules slurry as defined above, wherein the oil comprises a perfume;
(ii) at least one ingredient selected from the group consisting of a perfumery carrier, a perfumery co-ingredient and mixtures thereof;
(iii) optionally at least one perfumery adjuvant.

As liquid perfumery carriers one may cite, as non-limiting examples, an emulsifying system, i.e. a solvent and a surfactant system, or a solvent commonly used in perfumery. A detailed description of the nature and type of solvents commonly used in perfumery cannot be exhaustive. However, one can cite as non-limiting examples solvents such as dipropyleneglycol, diethyl phthalate, isopropyl myristate, benzyl benzoate, 2-(2-ethoxyethoxy)-1-ethanol or ethyl citrate, which are the most commonly used. For the compositions which comprise both a perfumery carrier and a perfumery co-ingredient, other suitable perfumery carriers than those previously specified, can be also ethanol, water/ethanol mixtures, limonene or other terpenes, isoparaffins such as those known under the trademark Isopar^{®} (origin: Exxon Chemical) or glycol ethers and glycol ether esters such as those known under the trademark Dowanol^{®} (origin: Dow Chemical Company). By "perfumery co-ingredient" it is meant here a compound, which is used in a perfuming preparation or a composition to impart a hedonic effect and which is not a microcapsule as defined above. In other words such a co-ingredient, to be considered as being a perfuming one, must be recognized by a person skilled in the art as being able to at least impart or modify in a positive or pleasant way the odor of a composition, and not just as having an odor.
The nature and type of the perfuming co-ingredients present in the perfuming composition do not warrant a more detailed description here, which in any case would not be exhaustive, the skilled person being able to select them on the basis of his general knowledge and according to the intended use or application and the desired organoleptic effect. In general terms, these perfuming co-ingredients belong to chemical classes as varied as alcohols, lactones, aldehydes, ketones, esters, ethers, acetates, nitriles, terpenoids, nitrogenous or sulphurous heterocyclic compounds and essential oils, and said perfuming co-ingredients can be of natural or synthetic origin. Many of these co-ingredients are in any case listed in reference texts such as the book by S. Arctander, Perfume and Flavor Chemicals, 1969, Montclair, New Jersey, USA, or its more recent versions, or in other works of a similar nature, as well as in the abundant patent literature in the field of perfumery. It is also understood that said co-ingredients may also be compounds known to release in a controlled manner various types of perfuming compounds also known as properfume or profragrance. Non-limiting examples of suitable properfumes may include 4-(dodecylthio)-4-(2,6,6-trimethyl-2-cyclohexen-1-yl)-2-butanone, 4-(dodecylthio)-4-(2,6,6-trimethyl-1-cyclohexen-1-yl)-2-butanone, trans-3-(dodecylthio)-1-(2,6,6-trimethyl-3-cyclohexen-1-yl)-1-butanone, 2-(dodecylthio)octan-4-one, 2-phenylethyl oxo(phenyl)acetate, 3,7-dimethylocta-2,6-dien-1-yl oxo(phenyl)acetate, (Z)-hex-3-en-1-yl oxo(phenyl)acetate, 3,7-dimethyl-2,6-octadien-1-yl hexadecanoate, bis(3,7-dimethylocta-2,6-dien-1-yl) succinate, (2-((2-methylundec-1-en-1-yl)oxy)ethyl)benzene, 1-methoxy-4-(3-methyl-4-phenethoxybut-3-en-1-yl)benzene, (3-methyl-4-phenethoxybut-3-en-1-yl)benzene, 1-(((Z)-hex-3-en-1-yl)oxy)-2-methylundec-1-ene, (2-((2-methylundec-1-en-1-yl)oxy)ethoxy)benzene, 2-methyl-1-(octan-3-yloxy)undec-1-ene, 1-methoxy-4-(1-phenethoxyprop-1-en-2-yl)benzene, 1-methyl-4-(1-phenethoxyprop-1-en-2-yl)benzene, 2-(1-phenethoxyprop-1-en-2-yl)naphthalene, (2-phenethoxyvinyl)benzene, 2-(1-((3,7-dimethyloct-6-en-1-yl)oxy)prop-1-en-2-yl)naphthalene, (2-((2-pentylcyclopentylidene)methoxy)ethyl)benzene, 4-allyl-2-methoxy-1-((2-methoxy-2-phenylvinyl)oxy)benzene, (2-((2-heptylcyclopentylidene)methoxy)ethyl)benzene, 1-isopropyl-4-methyl-2-((2-pentylcyclopentylidene)methoxy)benzene, 2-methoxy-1-((2-pentylcyclopentylidene)methoxy)-4-propylbenzene, 3-methoxy-4-((2-methoxy-2-phenylvinyl)oxy)benzaldehyde, 4-((2-(hexyloxy)-2-phenylvinyl)oxy)-3-methoxybenzaldehyde or a mixture thereof.

By "perfumery adjuvant" we mean here an ingredient capable of imparting additional added benefit such as a color, a particular light resistance, chemical stability, etc. A detailed description of the nature and type of adjuvant commonly used in perfuming bases cannot be exhaustive, but it has to be mentioned that said ingredients are well known to a person skilled in the art.

Preferably, the perfuming composition according to the invention comprises between 0.01 and 30 % by weight of microcapsules as defined above.

The invention's microcapsules can advantageously be used in many application fields and used in consumer products. Microcapsules can be used in liquid form applicable to liquid consumer products as well as in powder form, applicable to powder consumer products.

According to a particular embodiment, the consumer product as defined above is liquid and comprises:
a) from 2 to 65% by weight, relative to the total weight of the consumer product, of at least one surfactant;
b) water or a water-miscible hydrophilic organic solvent; and
c) a microcapsule slurry as defined above,
d) optionally non-encapsulated perfume.

According to a particular embodiment, the consumer product as defined above is in a powder form and comprises:
a) from 2 to 65% by weight, relative to the total weight of the consumer product, of at least one surfactant;
b) a microcapsule powder as defined above.
c) optionally perfume powder that is different from the microcapsules defined above.

In the case of microcapsules including a perfume oil-based core, the products of the invention, can in particular be of used in perfumed consumer products such as product belonging to fine fragrance or "functional" perfumery. Functional perfumery includes in particular personal-care products including hair-care, body cleansing, skin care, hygiene-care as well as home-care products including laundry care, surface care and air care. Consequently, also disclosed is a perfumed consumer product comprising as a perfuming ingredient, the microcapsules defined above or a perfuming composition as defined above. The perfume element of said consumer product can be a combination of perfume microcapsules as defined above and free or non-encapsulated perfume, as well as other types of perfume microcapsules than those here-disclosed.

In particular a liquid consumer product comprising:
a) from 2 to 65% by weight, relative to the total weight of the consumer product, of at least one surfactant;
b) water or a water-miscible hydrophilic organic solvent; and
c) a perfuming composition as defined above is another object of the invention.

Also a powder consumer product comprising
(a) from 2 to 65% by weight, relative to the total weight of the consumer product, of at least one surfactant; and
(b) a perfuming composition as defined above is part of the invention.

The invention's microcapsules can therefore be added as such or as part of a disclosed perfuming composition in a perfumed consumer product.

For the sake of clarity, it has to be mentioned that, by "perfumed consumer product" it is meant a consumer product which is expected to deliver among different benefits a perfuming effect to the surface to which it is applied (e.g. skin, hair, textile, paper, or home surface) or in the air (air-freshener, deodorizer etc.). In other words, a perfumed consumer product according to the invention is a manufactured product which comprises a functional formulation also referred to as "base", together with benefit agents, among which an effective amount of microcapsules according to the invention.

The nature and type of the other constituents of the perfumed consumer product do not warrant a more detailed description here, which in any case would not be exhaustive, the skilled person being able to select them on the basis of his general knowledge and according to the nature and the desired effect of said product. Base formulations of consumer products in which the microcapsules of the invention can be incorporated can be found in the abundant literature relative to such products. These formulations do not warrant a detailed description here which would in any case not be exhaustive. The person skilled in the art of formulating such consumer products is perfectly able to select the suitable components on the basis of his general knowledge and of the available literature.

Non-limiting examples of suitable perfumed consumer products can be a perfume, such as a fine perfume, a cologne, an after-shave lotion, a body-splash; a fabric care product, such as a liquid or solid detergent, tablets and unit dose (single or multi chambers), a fabric softener, a dryer sheet, a fabric refresher, an ironing water, or a bleach; a personal-care product, such as a hair-care product (e.g. a shampoo, hair conditioner, a coloring preparation or a hair spray), a cosmetic preparation (e.g. a vanishing cream, body lotion or a deodorant or antiperspirant), or a skin-care product (e.g. a perfumed soap, shower or bath mousse, body wash, oil or gel, bath salts, or a hygiene product); an air care product, such as an air freshener or a "ready to use" powdered air freshener; or a home care product, such all-purpose cleaners, liquid or power or tablet dishwashing products, toilet cleaners or products for cleaning various surfaces, for example sprays & wipes intended for the treatment / refreshment of textiles or hard surfaces (floors, tiles, stone-floors etc.); a hygiene product such as sanitary napkins, diapers, toilet paper.

Another object of the invention is a consumer product comprising:
- a personal care active base, and
- microcapsules as defined above,
wherein the consumer product is in the form of a personal care composition.

Personal care active bases in which the microcapsules of the invention can be incorporated can be found in the abundant literature relative to such products. These formulations do not warrant a detailed description here which would in any case not be exhaustive. The person skilled in the art of formulating such consumer products is perfectly able to select the suitable components on the basis of his general knowledge and of the available literature.
The personal care composition is preferably chosen in the group consisting of a hair-care product (e.g. a shampoo, hair conditioner, a coloring preparation or a hair spray), a cosmetic preparation (e.g. a vanishing cream, body lotion or a deodorant or antiperspirant), or a skin-care product (e.g. a perfumed soap, shower or bath mousse, body wash, oil or gel, bath salts, or a hygiene product);

Another object of the invention is a consumer product comprising:
- a home care or a fabric care active base, and
- microcapsules as defined above, wherein the consumer product is in the form of a home care or a fabric care composition.

Home care or fabric care active bases in which the microcapsules of the invention can be incorporated can be found in the abundant literature relative to such products. These formulations do not warrant a detailed description here which would in any case not be exhaustive. The person skilled in the art of formulating such consumer products is perfectly able to select the suitable components on the basis of his general knowledge and of the available literature.

Preferably, the consumer product comprises from 0.1 to 15 wt%, more preferably between 0.2 and 5 wt% of the microcapsules of the present invention, these percentages being defined by weight relative to the total weight of the consumer product. Of course the above concentrations may be adapted according to the benefit effect desired in each product.

For liquid consumer product mentioned below, by "active base", it should be understood that the active base includes active materials (typically including surfactants) and water.

For solid consumer product mention below, by "active base", it should be understood that the active base includes active materials (typically including surfactants) and auxiliary agents (such as bleaching agents, buffering agent; builders; soil release or soil suspension polymers; granulated enzyme particles, corrosion inhibitors, antifoaming, sud suppressing agents; dyes, fillers, and mixtures thereof).

### Fabric softener

Also disclosed is a consumer product in the form of a fabric softener composition comprising:
- a fabric softener active base; preferably comprising at least one active material chosen in the group consisting of dialkyl quaternary ammonium salts, dialkyl ester quaternary ammonium salts (esterquats), Hamburg esterquat (HEQ), TEAQ (triethanolamine quat), silicones and mixtures thereof, the active base being used preferably in an amount comprised between 85 and 99.95% by weight based on the total weight of the composition,
- microcapsules or a microcapsule slurry as defined above, preferably in an amount comprised between 0.05 to 15 wt%, more preferably between 0.1 and 5 wt% by weight based on the total weight of the composition,
- optionally free perfume oil.

### Liquid detergent

Also disclosed is a consumer product in the form of a liquid detergent composition comprising:
- a liquid detergent active base; preferably comprising at least one active material chosen in the group consisting of anionic surfactant such as alkylbenzenesulfonate (ABS), secondary alkyl sulfonate (SAS), primary alcohol sulfate (PAS), lauryl ether sulfate (LES), methyl ester sulfonate (MES) and nonionic surfactant such as alkyl amines, alkanolamide, fatty alcohol poly(ethylene glycol) ether, fatty alcohol ethoxylate (FAE), ethylene oxide (EO) and propylene oxide (PO) copolymers, amine oxydes, alkyl polyglucosides, alkyl polyglucosamides, the active base being used preferably in an amount comprised between 85 and 99.95% by weight based on the total weight of the composition,
- microcapsules or a microcapsule slurry as defined above, preferably in an amount comprised between 0.05 to 15 wt%, more preferably between 0.1 and 5 wt% by weight based on the total weight of the composition,
- optionally free perfume oil.

### Solid detergent

Also disclosed is a consumer product in the form of a solid detergent composition comprising:
- a solid detergent active base; preferably comprising at least one active material chosen in the group consisting of anionic surfactant such as alkylbenzenesulfonate (ABS), secondary alkyl sulfonate (SAS), primary alcohol sulfate (PAS), lauryl ether sulfate (LES), methyl ester sulfonate (MES) and nonionic surfactant such as alkyl amines, alkanolamide, fatty alcohol poly(ethylene glycol) ether, fatty alcohol ethoxylate (FAE), ethylene oxide (EO) and propylene oxide (PO) copolymers, amine oxydes, alkyl polyglucosides, alkyl polyglucosamides, the active base being used preferably in an amount comprised between 85 and 99.95% by weight based on the total weight of the composition,
- a microcapsule powder or microcapsule slurry as defined above, preferably in an amount comprised between 0.05 to 15 wt%, more preferably between 0.1 and 5 wt% by weight based on the total weight of the composition,
- optionally free perfume oil.

Also disclosed is a consumer product in the form of a shampoo or a shower gel composition comprising:
- a shampoo or a shower gel active base; preferably comprising at least one active material chosen in the group consisting of sodium alkylether sulfate, ammonium alkylether sulfates, alkylamphoacetate, cocamidopropyl betaine, cocamide MEA, alkylglucosides and aminoacid based surfactants and mixtures thereof, the active base being used preferably in an amount comprised between 85 and 99.95% by weight based on the total weight of the composition,
- microcapsules or a microcapsule slurry as defined above, preferably in an amount comprised between 0.05 to 15 wt%, more preferably between 0.1 and 5 wt% by weight based on the total weight of the composition,
- optionally free perfume oil.

### Rinse-Off Conditioner

Also disclosed is a consumer product in the form of a rinse-off conditioner composition comprising:
- a rinse-off conditioner active base; preferably comprising at least one active material chosen in the group consisting of cetyltrimonium chloride, stearyl trimonium chloride, benzalkonium chloride, behentrimonium chloride and mixture thereof, the active base being used preferably in an amount comprised between 85 and 99.95% by weight based on the total weight of the composition,
- microcapsules or a microcapsule slurry as defined above, preferably in an amount comprised between 0.05 to 15 wt%, more preferably between 0.1 and 5 wt% by weight based on the total weight of the composition,
- optionally free perfume oil.

### Solid scent booster

disclosed is a consumer product in the form of a solid scent booster composition comprising:
- a solid carrier, preferably chosen in the group consisting of urea, sodium chloride, sodium sulphate, sodium acetate, zeolite, sodium carbonate, sodium bicarbonate, clay, talc, calcium carbonate, magnesium sulfate, gypsum, calcium sulfate, magnesium oxide, zinc oxide, titanium dioxide, calcium chloride, potassium chloride, magnesium chloride, zinc chloride, saccharides such as sucrose, mono-, di-, and polysaccharides and derivatives such as starch, cellulose, methyl cellulose, ethyl cellulose, propyl cellulose, polyols/sugar alcohols such as sorbitol, maltitol, xylitol, erythritol, and isomalt, PEG, PVP, citric acid or any water soluble solid acid, fatty alcohols or fatty acids and mixtures thereof,
- microcapsules or a microcapsule slurry as defined above, in a powdered form, preferably in an amount comprised between 0.05 to 15 wt%, more preferably between 0.1 and 5 wt% by weight based on the total weight of the composition,
- optionally free perfume oil.

### Liquid scent booster

Also disclosed is a consumer product in the form of a liquid scent booster composition comprising:
- an aqueous phase,
- a surfactant system essentially consisting of one or more than one non-ionic surfactant, wherein the surfactant system has a mean HLB between 10 and 14, preferably chosen in the group consisting of ethoxylated aliphatic alcohols, POE/PPG (polyoxyethylene and polyoxypropylene) ethers, mono and polyglyceryl esters, sucrose ester compounds, polyoxyethylene hydroxylesters, alkyl polyglucosides, amine oxides and combinations thereof;
- a linker chosen in the group consisting of alcohols, salts and esters of carboxylic acids, salts and esters of hydroxyl carboxylic acids, fatty acids, fatty acid salts, glycerol fatty acids, surfactant having an HLB less than 10 and mixtures thereof, and

- microcapsules or a microcapsule slurry as defined above, in the form of a slurry, preferably in an amount comprised between 0.05 to 15 wt%, more preferably between 0.1 and 5 wt% by weight based on the total weight of the composition,
- optionally free perfume oil.

### Hair coloration

Also disclosed is a consumer product in the form of an oxidative hair coloring composition comprising:
- an oxidizing phase comprising an oxidizing agent and an alkaline phase comprising an alkakine agent, a dye precursor and a coupling compound; wherein said dye precursor and said coupling compound form an oxidative hair dye in the presence of the oxidizing agent, preferably in an amount comprised between 85 and 99.95% by weight based on the total weight of the composition,
- microcapsules or a microcapsule slurry as defined above, preferably in an amount comprised between 0.05 to 15 wt%, more preferably between 0.1 and 5 wt% by weight based on the total weight of the composition,
- optionally free perfume oil

### Perfuming composition

According to a particular embodiment, the consumer product is in the form of a perfuming composition comprising:
- 0.1 to 30%, preferably 0.1 to 20% of microcapsules or microcapsules slurry as defined previously,
- 0 to 40%, preferably 3-40% of perfume, and
- 20-90%, preferably 40-90% of ethanol, by weight based on the total weight of the perfuming composition.

The invention will now be further described by way of examples. It will be appreciated that the invention as claimed is not intended to be limited in any way by these examples.

### EXAMPLES

### Example 1

### Cellulose acetoacetate suspension preparation

1/ Cellulose powder (1.0g) was dispersed into ionic liquids (1-allyl-3-methylimidazolium chloride (AMIMCl) (19g)). Then, the mixture system was stirred at 90 °C until cellulose was dissolved completely.
2/ Dimethylformamide (DMF) (20.0 mL) was added to the cellulose solution and heated to 110 °C. Tert-butyl acetoacetate (t-BAA, 6 g, 37.9 mmol) was added dropwise to the reaction mixture under nitrogen with magnetic stirring for 1 h. The reaction was ended when the water solubility was good.
3/ After the reaction, the reaction system was cooled to 30 °C, and the liquid in the flask was slowly added to a large amount of ethanol to obtain a large amount of pale yellow precipitation.
4/ The precipitation was washed with ethanol and filtered until the purified cellulose acetoacetate was obtained. And then the powder was dried at 60 °C in vacuum for 24 h.

### Example 2

### Starch acetoacetate suspension preparation

1/ Corn starch (10.0g) was dispersed into 140 ml dimethyl sulfoxide (DMSO), then the mixture system was stirred at 90 °C until starch was dissolved completely.
2/ The solution was heated to 110 °C and tert-butyl acetoacetate (t-BAA, 41.1 g) was added dropwise to the reaction mixture under the protection of nitrogen with magnetic stirring for 4h.
3/ After the reaction, the products were precipitated with ethanol, then washed with ethanol, and dried at 60 °C in vacuum for 24 h.

### Example 3

### Microcapsules preparation with cellulose acetoacetate

The cellulose acetoacetate (CAA) obtained in example 1 was dissolved into deionized water as water phase. Water phase can comprise optionally a polyfunctional monomer. A perfume oil formulation containing at least one polyfunctional monomer was prepared to form an oil phase.

The weight ratio of oil/water was controlled at 3/7.
i) Stable emulsion was formed by mixing the oil phase and the water phase with a homogenizer.
ii) The emulsion was transferred into a reactor and the interfacial reaction was carried out at 80°C for 3h.

Optionally, catalyst(s) were added in the oil phase or in the emulsion.

The composition of microcapsule samples is shown in Table 1.

**Table 1: Microcapsule composition**

| **Sample** | **Cellulose acetoacetate** | **Perfume oil ^{a)}** | **Polyfunctional monomer** | **Catalyst** |
|---|---|---|---|---|
| A | 0.7% | 29.7% | 0.3% 1,12-Diaminododecane in oil phase | / |
| B | 0.7% | 28.2% | 0.3% 1,12-Diaminododecane in oil phase + 1.5% Vinyltriethoxysilane in oil phase | / |
| C | 0.7% | 26.8% | 3.0% Ethylene glycol dimethacrylate in oil phase | 0.1% Benzoyl peroxide (added in the oil phase) + 0.1% 1,8-Diazabicyclo[5.4.0]undec-7-ene (added in the oil phase) |
| D | 0.7% | 25.3% | 3.0% Ethylene glycol dimethacrylate in oil phase + 1.5% Vinyltriethoxysilane in oil phase | 0.1% Benzoyl peroxide (added in the oil phase) + 0.1% 4-Dimethylaminopyridine (added in the emulsion) |
| E | 0.7% | 28.5% | 1.5% (3-Aminopropyl) triethoxysilane in oil phase | / |
| F | 0.7% | 29.1% | 0.9% Takenate^{®} D-110N ^{b)} in oil phase | / |
| G | 0.7% | 28.1% | 0.9% Takenate^{®} D-110N ^{b)} in oil phase + 3.5% Melamine-glyoxal resin ^{c)} in water phase | / |

| | | | | |
|---|---|---|---|---|
| a) See Table 3 b) trimethylol propane adduct of xylylene diisocyanate; origin: Mitsui Chemicals, 75% polyisocyanate /25% ethyl acetate c) reaction of Melamine, Glyoxal and 2,2-Dimethoxyacetaldehyde | | | | |

### Example 4

### Microcapsules preparation with starch acetoacetate

The stabilizer was dissolved into deionized water as water phase. A perfume oil formulation containing at least one polyfunctional monomer was prepared to form an oil phase. The weight ratio of oil/water was controlled at 3/7.
i) Stable emulsion was formed by mixing the oil phase and the water phase with a homogenizer.
ii) Starch acetoacetate was added into the emulsion and the interfacial reaction was carried out at 80°C for 3h.

Optionally, catalyst(s) were added in the oil phase or in the emulsion.

The composition of microcapsule samples is shown in Table 2.

**Table 2: Microcapsule composition**

| **Sample** | **Stabilizer** | **Starch acetoacetate** | **Perfume oil ^{a)}** | **Polyfunctional monomer** | **Catalyst** |
|---|---|---|---|---|---|
| H | 0.95% Gum Arabic ^{c)} | 0.2% | 26.4% | 0.9% Takenate^{®} D-110N ^{b)} in oil phase | / |
| I | 0.81% Gum Arabic | 0.2% | 29.1% | 2.3% Ethylene glycol dimethacrylate in oil phase | 0.12% Ammonium persulfate + 0.04% Sodium hydrogen sulfite (added in the emulsion) |

| | | | | | |
|---|---|---|---|---|---|
| a) See Table 3 b) trimethylol propane adduct of xylylene diisocyanate; origin: Mitsui Chemicals, 75% polyisocyanate /25% ethyl acetate c) Superstab^{™} (Origin: Nexira) | | | | | |

**Table 3 - Formulation of the perfume oil**

| **Ingredients** | **% in oil** |
|---|---|
| Ethyl 2-methyl-pentanoate | 3.20% |
| Eucalyptol | 7.80% |
| 2,4-Dimethyl-3-cyclohexene-1-carbaldehyde | 0.75% |
| Aldehyde C10 | 0.75% |
| Citronellyl Nitrile | 4.30% |
| Isobornyl acetate | 3.00% |
| 2-tert-butyl-1-cyclohexyl acetate | 9.80% |
| Citronellyl Acetate | 1.30% |
| 2-Methylundecanal | 3.00% |
| Diphenyloxide | 0.80% |
| Aldehyde C₁₂ | 1.30% |
| Dicyclopentadiene acetate | 9.85% |
| Ionone beta | 3.30% |
| Undecalactone gamma | 18.75% |
| Hexyl Salicylate | 15.90% |
| Benzyl Salicylate | 16.20% |

### Example 5

### Characterization of the microcapsules

**Size measurement:** Mean size (D [4,3]) of microcapsule slurry was measured by using the Mastersizer 3000 equipment from Malvern Instruments Ltd., UK

**Zeta potential measurement:** Zeta potential of microcapsules was examined using Zetasizer Nano ZS from Malvern Instruments Ltd., UK

**Oil permeability:** The permeability of encapsulated fragrance oil in microcapsule was monitored by a Thermogravimetric Analyzer (TGA/DSC 1, Mettler-Toledo) equipped with a microbalance having an accuracy of 1 µg. The weight loss of encapsulated fragrance oil was monitored at 50°C for 4hrs.

**Table 4 - Microcapsule characterization**

| Example | Mean size (µm) | Zeta potential (mV) | Ratio of lost perfume oil/encapsulated perfume oil under TGA test % |
|---|---|---|---|
| A | 43.3 | 13.1 ± 3.1 | 6.84 |
| B | 32.1 | -0.3 ± 0.4 | 1.45 |
| C | 31.5 | -8.1 ± 0.7 | 3.15 |
| D | 27.9 | -13.8 ± 1.3 | 3.18 |
| E | 18.3 | -3.21±0.1 mV | 8.78 |
| F | 46.6 | - 1. 13±0.1 mV | 4.46 |
| | | | |
| G | 50 | -6.12±0.3 mV | 8.59 |
| H | 11.9 | -24.5±1.4mV | 1.94 |
| I | 53.7 | -22.8±0.7 mV | 7.83 |

### Example 6

### Fabric softener composition

Microcapsules A-I of the present invention are dispersed in a fabric softener composition described in Table 5 to obtain a concentration of encapsulated perfume oil at 0.22%.

**Table 5: Fabric Conditioner composition**

| Product | Wt % |
|---|---|
| Stepantex VL 90A | 8.88 |
| Calcium Chloride Sol. 10% | 0.36 |
| Proxel GXL | 0.04 |
| Perfume | 1.00 |
| Water | 89.72 |
| TOTAL | 100 |

### Example 7

### Liquid detergent composition

Microcapsules A-I of the present invention are dispersed in a liquid detergent base described in Table 6 to obtain a concentration of encapsulated perfume oil at 0.22%.

**Table 6 - Liquid detergent composition**

| Ingredients | Concentration [wt%] |
|---|---|
| Sodium C14-17 Alkyl Sec Sulfonate¹⁾ | 7 |
| Fatty acids, C12-18 and C18-unsaturated²⁾ | 7.5 |
| C12/14 fatty alcohol polyglycol ether with 7 mol EO³⁾ | 17 |
| Triethanolamine | 7.5 |
| Propylene Glycol | 11 |
| Citric acid | 6.5 |
| Potassium Hydroxyde | 9.5 |
| Protease | 0.2 |
| Amylase | 0.2 |
| Mannanase | 0.2 |
| Acrylates/Steareth-20 Methacrylate structuring Crosspolymer⁴⁾ | 6 |
| Deionized Water | 27.4 |

| | |
|---|---|
| 1) Hostapur SAS 60; Origin: Clariant 2) Edenor K 12-18; Origin: Cognis 3) Genapol LA 070; Origin: Clariant 4) Aculyn 88; Origin: Dow Chemical | |

### Example 8

### Rinse-off conditioner

Microcapsules A-I of the present invention are dispersed in a rinse-off conditioner base described in table 7 to obtain a concentration of encapsulated perfume oil at 0.5%.

**Table 7 - Rinse-off conditioner composition**

| | **Ingredients** | **Concentration [wt%]** |
|---|---|---|
| **A** | Water deionized | **81.8** |
| | Behentrimonium Chloride ¹⁾ | **2.5** |
| | Hydroxyethylcellulose ²⁾ | **1.5** |
| | | |
| **B** | Cetearyl Alcohol ³⁾ | **4** |
| | Glyceryl Stearate (and) PEG-100 Stearate ⁴⁾ | **2** |
| | Behentrimonium Methosulfate (and) Cetyl alcohol (and) Butylene Glycol ⁵⁾ | **4** |
| | Ethoxy (20) Stearyl Alcohol ⁶⁾ | **1** |
| | | |
| **C** | Amodimethicone (and) Trideceth-12 (and) Cetrimonium Chloride⁷⁾ | **3** |
| | Chlorhexidine Digluconate ⁸⁾ 20% aqueous solution | **0.2** |
| **D** | Citric acid 10% aqueous sol. till pH 3.5-4 | **q.s.** |
| | **TOTAL:** | **100** |

| | | |
|---|---|---|
| 1) Genamin KDM P, Clariant 2) Tylose H10 Y G4, Shin Etsu 3) Lanette O, BASF 4) Arlacel 165-FP-MBAL-PA-(RB), Croda 5) Incroquat Behenyl TMS-50-MBAL-PA-(MH) HA4112, Croda 6) SP Brij S20 MBAL-PA(RB), Croda 7) Xiameter DC MEM-0949 Emulsion, Dow Corning 8) Alfa Aesar | | |

### Example 9

### Shampoo composition

Microcapsules A-I of the present invention are weighed and mixed in a shampoo composition to add the equivalent of 0.2% perfume.

**Table 8 - Shampoo composition**

| | **Ingredients** | **Concentration [wt%]** |
|---|---|---|
| **A** | Water deionized | **44.4** |
| | Polyquaternium-10 ¹⁾ | **0.3** |
| | Glycerin 85% ²⁾ | **1** |
| | DMDM Hydantoin ³⁾ | **0.2** |
| | | |
| **B** | Sodium Laureth Sulfate ⁴⁾ | **28** |
| | Cocamidopropyl Betaine ⁵⁾ | **3.2** |
| | Disodium Cocoamphodiacetate ⁶⁾ | **4** |
| | Ethoxy (20) Stearyl Alcohol ⁶⁾ | **1** |
| | | |
| **C** | Sodium Laureth Sulfate ⁴⁾ | **3** |
| | Glyceryl Laureate ⁷⁾ | **0.2** |
| | | |
| **D** | Water deionized | **1** |
| | Sodium Methylparaben ⁸⁾ | **0.1** |
| | | |
| **E** | Sodium Chloride 10% aqueous sol. | **15** |
| | Citric acid 10% aqueous sol. till pH 5.5-6 | **q.s.** |
| | Perfume | **0.5** |
| | **TOTAL:** | **100** |

| | | |
|---|---|---|
| 1) Ucare Polymer JR-400, Noveon 2) Schweizerhall 3) Glydant, Lonza 4) Texapon NSO IS, Cognis 5) Tego Betain F 50, Evonik 6) Amphotensid GB 2009, Zschimmer & Schwarz 7) Monomuls 90 L-12, Gruenau 8) Nipagin Monosodium, NIPA | | |

### Example 10

### Antiperspirant roll-on emulsion composition

Microcapsules A-I of the present invention are weighed and mixed in antiperspirant roll-on emulsion composition to add the equivalent of 0.2% perfume.

**Table 9 - Antiperspirant composition**

| **Ingredient** | **Amount (wt %)** |
|---|---|
| Steareth-2¹⁾ (Part A) | 3.25 |
| Steareth-21²⁾ (Part A) | 0.75 |
| PPG-15 Stearyl Ether³⁾ (Part A) | 4 |
| WATER deionised (Part B) | 51 |
| Aluminum Chlorohydrate 50% aqueous solution⁴⁾ (Part C) | 40 |
| Fragrance (Part D) | 1 |

| | |
|---|---|
| 1) BRIJ 72; origin : ICI 2) BRIJ 721; origin : ICI 3) ARLAMOL E; origin : UNIQEMA-CRODA 4) LOCRON L; origin : CLARIAN | |

Part A and B are heated separately to 75°C; Part A is added to Part B under stirring and the mixture is homogenized for 10 min. Then, the mixture is cooled under stirring; and Part C is slowly added when the mixture reached 45°C and Part D when the mixture reached at 35 °C while stirring. Then the mixture is cooled to room temperature.

### Example 11

### Shower-gel composition

Microcapsules A-I of the present invention are weighed and mixed in the following composition to add the equivalent of 0.2% perfume.

**Table 10 - Shower gel composition**

| **Ingredients** | **Amount (% wt)** | **Function** |
|---|---|---|
| WATER deionized | 49.350 | Solvent |
| Tetrasodium EDTA ¹⁾ | 0.050 | Chelating agent |
| Acrylates Copolymer²⁾ | 6.000 | Thickener |
| Sodium C12-C15 Pareth Sulfate ³⁾ | 35.000 | Surfactant |
| Sodium Hydroxide 20% aqueous solution | 1.000 | pH adjuster |
| Cocamidopropyl Betaine⁴⁾ | 8.000 | Surfactant |
| Methylchloroisothiazolinone and Methylisothiazolinone⁵⁾ | 0.100 | Preservative |
| Citric Acid (40%) | 0.500 | pH adjuster |

| | | |
|---|---|---|
| 1) EDETA B POWDER; trademark and origin: BASF 2) CARBOPOL AQUA SF-1 POLYMER; trademark and origin: NOVEON 3) ZETESOL AO 328 U; trademark and origin: ZSCHIMMER & SCHWARZ 4) TEGO-BETAIN F 50; trademark and origin: GOLDSCHMIDT 5) KATHON CG; trademark and origin: ROHM & HASS. | | |

## Claims

1. A core-shell microcapsule comprising:
a) an oil-based core comprising a hydrophobic material, preferably a perfume oil; and
b) a polymeric shell comprising an acetoacetyl polymer,
wherein the acetoacetyl polymer is not an acetoacetyl polyvinyl alcohol (PVOH) or an acetoacetyl modified polyvinyl alcohol (PVOH).

2. The microcapsule according to claim 1, wherein the polymeric shell is made of a polymeric material selected from the group consisting of polyenamine, polyurea, polyurethane, polyamide, polyacrylate, polysiloxane, polycarbonate, polysulfonamide, urea formaldehyde, melamine formaldehyde resin, melamine urea resin, melamine glyoxal resin, gelatin/gum arabic and mixtures thereof.

3. The microcapsule according to anyone of the preceding claims, wherein the acetoacetyl polymer is selected from the group consisting of cellulose acetoacetate, starch acetoacetate, polyfunctional acetoacetate with two to six acetoacetate functional groups, and mixtures thereof.

4. The microcapsule according to anyone of the preceding claims, wherein the polymeric shell comprises less than 20% by weight of a polymeric material based on the total weight of the microcapsule.

5. The microcapsule according to anyone of the preceding claims, wherein the hydrophobic material comprises a perfume oil.

6. A process for preparing core-shell microcapsules as defined in anyone of claims 1-5, wherein the process comprises the steps of:
1) preparing a water phase;
2) preparing an oil phase comprising a hydrophobic material, preferably a perfume oil;
3) adding the oil phase to the water phase and mixing them to form an oil-in-water emulsion, under conditions allowing the formation of core-shell microcapsule by interfacial polymerization and/or interfacial reaction,
wherein a polyfunctional monomer is added in step 1) in the water phase and/or in step 2) in the oil phase and/or in step 3) in the emulsion, and
wherein an acetoacetyl polymer is added in the water phase and/or in the oil phase and/or in the emulsion.

7. The process according to claim 6, wherein the polyfunctional monomer is chosen in the group consisting of at least polyamine, polyisocyanate, poly anhydride, poly maleic anhydride, poly acid chloride, polyepoxide, acrylate monomers or (meth)acrylate monomers, polyalkoxysilane or alkoxysilane, melamine-based resin, and mixtures thereof.

8. The process according to claim 6 or 7, wherein the acetoacetyl polymer is obtained the following process:
(i) dissolving a polymer having hydroxyl groups into a solvent to form a homogeneous solution
(ii) inducing acetoacetylation of said polymer via reaction with the hydroxyl groups
(iii) inducing precipitation of the polymer from the solution obtained in step (ii),
(iv) Optionally, purifying the precipitated, preferably by a washing process, to obtain an acetoacetyl polymer.

9. The process according to any one of claims 6-8, wherein a reactant is added in step 1) and/or step 3).

10. The process according to any one of claims 6-9, wherein the total amount of acetoacetyl polymer is comprised between 0.01 and 10 wt%, preferably between 0.01and 5 wt% based on the total weight of the emulsion.

11. The process according to any one of claims 6-10, **characterized in that** the oil phase represents between 5 and 60%, preferably between 20 and 40% by weight of the emulsion.

12. A consumer product comprising:
- a personal care active base, and
- microcapsules as defined in claims 1-5,
wherein the consumer product is in the form of a personal care composition.

13. A consumer product comprising:
- a home care or a fabric care active base, and
- microcapsules as defined in claims 1-5,
wherein the consumer product is in the form of a home care or a fabric care composition.

## Patentansprüche

1. Kern-Hülle-Mikrokapsel, umfassend:
a) einen ölbasierten Kern, der ein hydrophobes Material, vorzugsweise ein Parfümöl, umfasst; und
b) eine polymere Hülle, die ein Acetoacetylpolymer umfasst,
wobei das Acetoacetylpolymer kein Acetoacetyl-Polyvinylalkohol (PVOH) oder Acetoacetylmodifizierter Polyvinylalkohol (PVOH) ist.

2. Mikrokapsel nach Anspruch 1, wobei die polymere Hülle aus einem polymeren Material gefertigt ist, ausgewählt aus der Gruppe bestehend aus Polyenamin, Polyharnstoff, Polyurethan, Polyamid, Polyacrylat, Polysiloxan, Polycarbonat, Polysulfonamid, Harnstoff-Formaldehyd, MelaminFormaldehyd-Harz, Melamin-Harnstoff-Harz, Melamin-Glyoxal-Harz, Gelatine/Gummi arabicum und Gemische davon.

3. Mikrokapsel nach einem der vorstehenden Ansprüche, wobei das Acetoacetylpolymer ausgewählt ist aus der Gruppe bestehend aus Celluloseacetoacetat, Stärkeacetoacetat, polyfunktionellem Acetoacetat mit zwei bis sechs Acetoacetat-Funktionsgruppen und Gemische davon.

4. Mikrokapsel nach einem der vorstehenden Ansprüche, wobei die polymere Hülle weniger als 20 Gew.-% eines polymeren Materials, bezogen auf das Gesamtgewicht der Mikrokapsel, umfasst.

5. Mikrokapsel nach einem der vorstehenden Ansprüche, wobei das hydrophobe Material ein Parfümöl umfasst.

6. Verfahren zur Herstellung von Kern-Hülle-Mikrokapseln nach einem der Ansprüche 1-5, wobei das Verfahren die folgenden Schritte umfasst:
1) Vorbereiten einer Wasserphase;
2) Vorbereiten einer Ölphase, die ein hydrophobes Material, vorzugsweise ein Parfümöl, umfasst;
3) Zugeben der Ölphase zur Wasserphase und Mischen beider Phasen zum Bilden einer Ölin-Wasser-Emulsion unter Bedingungen, die die Bildung von Kern-Hülle-Mikrokapseln durch Grenzflächenpolymerisation und/oder Grenzflächenreaktion ermöglichen,
wobei ein polyfunktionelles Monomer in Schritt 1) in der Wasserphase und/oder in Schritt 2) in der Ölphase und/oder in Schritt 3) in der Emulsion zugegeben wird und
wobei ein Acetoacetylpolymer in der Wasserphase und/oder in der Ölphase und/oder in der Emulsion zugegeben wird.

7. Verfahren nach Anspruch 6, wobei das polyfunktionelle Monomer ausgewählt ist aus der Gruppe bestehend aus mindestens Polyamin, Polyisocyanat, Polyanhydrid, Polymaleinsäureanhydrid, Polysäurechlorid, Polyepoxid, Acrylatmonomeren oder (Meth)acrylatmonomeren, Polyalkoxysilan oder Alkoxysilan, Harz auf Melaminbasis und Gemische davon.

8. Verfahren nach Anspruch 6 oder 7, wobei das Acetoacetylpolymer durch das folgende Verfahren erhalten wird:
(i) Auflösen eines Polymers mit Hydroxylgruppen in einem Lösungsmittel zum Bilden einer homogenen Lösung
(ii) Einleiten einer Acetoacetylierung des Polymers durch Reaktion mit den Hydroxylgruppen
(iii) Einleiten einer Ausfällung des Polymers aus der in Schritt (ii) erhaltenen Lösung,
(iv) optional Reinigen des ausgefällten Produkts, vorzugsweise durch einen Waschprozess, um ein Acetoacetylpolymer zu erhalten.

9. Verfahren nach einem der Ansprüche 6-8, wobei in Schritt 1) und/oder Schritt 3) ein Reaktant zugegeben wird.

10. Verfahren nach einem der Ansprüche 6-9, wobei die Gesamtmenge des Acetoacetylpolymers zwischen 0,01 und 10 Gew.-%, vorzugsweise zwischen 0,01 und 5 Gew.-%, bezogen auf das Gesamtgewicht der Emulsion, liegt.

11. Verfahren nach einem der Ansprüche 6-10, **dadurch gekennzeichnet, dass** die Ölphase 5 bis 60%, vorzugsweise 20 bis 40 Gew.-% der Emulsion darstellt.

12. Verbraucherprodukt, umfassend:
- eine Aktivbasis für ein Körperpflegeprodukt und
- Mikrokapseln nach den Ansprüchen 1-5,
wobei das Verbraucherprodukt in der Form einer Körperpflegezusammensetzung vorliegt.

13. Verbraucherprodukt, umfassend:
- eine Aktivbasis für Haushaltspflege oder Textilpflege und
- Mikrokapseln nach den Ansprüchen 1-5,
wobei das Verbraucherprodukt in der Form eines Haushaltspflegeprodukts oder eines Textilpflegeprodukts vorliegt.

## Revendications

1. Microcapsule de type cœur-écorce comprenant :
a) un cœur à base d'huile comprenant un matériau hydrophobe, de préférence une huile parfumée ; et
b) une écorce polymère comprenant un polymère acétoacétyle,
dans laquelle le polymère acétoacétyle n'est pas un alcool polyvinylique (POVH) acétoacétyle ou un alcool polyvinylique (POVH) modifié par acétoacétyle.

2. Microcapsule selon la revendication 1, dans laquelle l'écorce polymère est constituée d'un matériau polymère choisi parmi le groupe consistant en polyénamine, polyurée, polyuréthane, polyamide, polyacrylate, polysiloxane, polycarbonate, polysulfonamide, résine urée-formaldéhyde, résine mélamine-formaldéhyde, résine mélamine-urée, résine mélamine-glyoxal, gélatine/gomme arabique et des mélanges de ceux-ci.

3. Microcapsule selon l'une quelconque des revendications précédentes, dans laquelle le polymère acétoacétyle est choisi parmi le groupe consistant en acétoacétate de cellulose, acétoacétate d'amidon, acétoacétate polyfonctionnel comportant deux à six groupes fonctionnels acétoacétate et des mélanges de ceux-ci.

4. Microcapsule selon l'une quelconque des revendications précédentes, dans laquelle l'écorce polymère comprend moins de 20 % en poids d'un matériau polymère par rapport au poids total de la microcapsule.

5. Microcapsule selon l'une quelconque des revendications précédentes, dans laquelle le matériau hydrophobe comprend une huile parfumée.

6. Processus de préparation de microcapsules de type cœur-écorce selon l'une quelconque des revendications 1-5, dans lequel le processus comprend les étapes de :
1) préparation d'une phase aqueuse ;
2) préparation d'une phase huileuse comprenant un matériau hydrophobe, de préférence une huile parfumée ;
3) ajout de la phase huileuse à la phase aqueuse et leur mélange pour former une émulsion huile-dans-eau, dans des conditions permettant la formation d'une microcapsule de type cœur-écorce par polymérisation interfaciale et/ou réaction interfaciale,
dans lequel un monomère polyfonctionnel est ajouté à l'étape 1) dans la phase aqueuse et/ou à l'étape 2) dans la phase huileuse et/ou à l'étape 3) dans l'émulsion, et
dans lequel un polymère acétoacétyle est ajouté dans la phase aqueuse et/ou dans la phase huileuse et/ou dans l'émulsion.

7. Processus selon la revendication 6, dans lequel le monomère polyfonctionnel est choisi parmi le groupe consistant au moins en polyamine, polyisocyanate, polyanhydride, polyanhydride maléique, polychlorure d'acide, polyépoxyde, monomères d'acrylate ou monomères de (méth)acrylate, polyalcoxysilane ou alcoxysilane, résine à base de mélamine et des mélanges de ceux-ci.

8. Processus selon la revendication 6 ou 7, dans lequel le polymère acétoacétyle est obtenu par le procédé suivant :
(i) dissolution d'un polymère présentant des groupes hydroxyle dans un solvant pour former une solution homogène
(ii) induction de l'acétoacétylation dudit polymère par réaction avec les groupes hydroxyle
(iii) induction de la précipitation du polymère à partir de la solution obtenue à l'étape (ii),
(iv) facultativement, purification du précipité, de préférence par un processus de lavage, afin d'obtenir un polymère acétoacétyle.

9. Processus selon l'une quelconque des revendications 6-8, dans lequel un réactif est ajouté à l'étape 1) et/ou à l'étape 3).

10. Processus selon l'une quelconque des revendications 6-9, dans lequel la quantité totale de polymère acétoacétyle est comprise entre 0,01 et 10 % en poids, de préférence entre 0,01 et 5 % en poids par rapport au poids total de l'émulsion.

11. Processus selon l'une quelconque des revendications 6-10, **caractérisé en ce que** la phase huileuse représente entre 5 et 60 %, de préférence entre 20 et 40 % en poids de l'émulsion.

12. Produit de consommation comprenant :
- une base active de soins personnels, et
- des microcapsules selon les revendications 1-5,
dans lequel le produit de consommation se présente sous la forme d'une composition de soins personnels.

13. Produit de consommation comprenant :
- une base active d'entretien ménager ou d'entretien de tissus, et
- des microcapsules selon les revendications 1-5,
dans lequel le produit de consommation se présente sous la forme d'une composition d'entretien ménager ou d'entretien de tissus.
